(19) Europäisches Patentamt
European Patent Office
Office européen des brevets

(11) **EP 2 184 304 A1**

(12) **EUROPÄISCHE PATENTANMELDUNG**

(43) Veröffentlichungstag:
**12.05.2010 Patentblatt 2010/19**

(51) Int Cl.:
*C08G 18/10* (2006.01)      *C08G 18/28* (2006.01)
*C08G 18/48* (2006.01)      *C09D 7/00* (2006.01)

(21) Anmeldenummer: **08155673.0**

(22) Anmeldetag: **06.05.2008**

(84) Benannte Vertragsstaaten:
**AT BE BG CH CY CZ DE DK EE ES FI FR GB GR
HR HU IE IS IT LI LT LU LV MC MT NL NO PL PT
RO SE SI SK TR**
Benannte Erstreckungsstaaten:
**AL BA MK RS**

(71) Anmelder: **BASF SE
67056 Ludwigshafen (DE)**

(72) Erfinder: **Die Erfindernennung liegt noch nicht vor**

(54) **Polyurethanverdicker**

(57)   Die vorliegende Erfindung betrifft neue Polyurethane sowie Mischungen solcher Polyurethane, Verfahren zu deren Herstellung, ihre Verwendung zur Herstellung von Wasser enthaltenden Zubereitungen und Zubereitungen, die die Polyurethane enthalten.

EP 2 184 304 A1

**Beschreibung**

Die vorliegende Erfindung betrifft Polyurethane (PU) sowie Mischungen solcher erfindungsgemäßer Polyurethane PU, Verfahren zur Herstellung von PU, die Verwendung von PU zur Herstellung von Wasser enthaltenden Zubereitungen und Zubereitungen die Polyurethane PU enthalten. Insbesondere betrifft die Erfindung in Wasser dispergierbares Polyurethan (PU) mit einem im Wesentlichen linearen Rückgrat aufgebaut aus alternierenden hydrophilen und hydrophoben Abschnitten, wobei

a. die beiden endständigen Abschnitte (T) hydrophob sind,
b. sich an jeden Abschnitt T je ein hydrophiler Abschnitt (S) direkt anschließt,
c. sich an jeden Abschnitt S mindestens ein hydrophober Abschnitt (D) auf mindestens einer Seite direkt anschließt, und
d. wobei mindestens ein hydrophiler Abschnitt (P) enthalten ist, wobei mindestens ein hydrophober Abschnitt D zwei Abschnitte P trennt, falls mehr als ein Abschnitt P enthalten ist,

und das Polyurethan mindestens drei hydrophile Abschnitte umfasst, und das Verhältnis der Molekulargewichte eines jeden hydrophilen Abschnittes S zu dem Molekulargewicht eines jeden hydrophilen Abschnittes P von 1 zu 1,4 bis 1 zu 140 beträgt, die mindestens zwei hydrophoben Abschnitte D aliphatische Diisocyanatreste sind, und der mindestens eine hydrophile Abschnitt P ein Polyetherrest mit einem Molekulargewicht von mindestens 1500 g/mol ist, oder eine Mischung unterschiedlicher Polyurethane PU.

Die vorliegende Erfindung umfasst des Weiteren Kombinationen der im Folgenden genannten Ausführungsformen.

Polyurethane sind Polymere, die beispielsweise durch Umsetzung von Alkoholen (z.B. Diolen) mit Diisocyanaten hergestellt werden können.

Seit Jahrzehnten werden diese Verbindungen in der Druckindustrie eingesetzt. Entsprechend der Wahl der Ausgangsstoffe und des stöchiometrischen Verhältnis der Ausgangsstoffe erhält man Polyurethane mit sehr unterschiedlichen physikalischchemischen Eigenschaften.

Erfindungsgemäße Polyurethane PU sind Polymere, die durch Umsetzung von Alkohol-alkoxylaten und/oder Polyetherpolyolen mit Isocyanaten oder Polyisocyanaten entstehen, und werden im Folgenden auch Polyetherurethane genannt.

Verdickungsmittel werden in großem Umfang zur Erhöhung der Viskosität von wässrigen Zubereitungen eingesetzt, beispielsweise auf den Gebieten Kosmetik, Human- und Tierernährung, Pharmazie und für Waschmittel, Farben und Lacke. Unter anderem sind auch Polyurethane als Verdickungsmittel bekannt.

Beispielsweise werden Polyurethanlösungen oder -dispersionen in wasserverdünnbarer wässriger oder überwiegend wässriger Phase vom Fachmann als HEUR-Verdicker bezeichnet (das Akronym HEUR leitet sich von "hydrophobically modified ethylene oxide urethan copolymer" ab), und bereits seit längerem in den unterschiedlichsten Anwendungsfeldern zur Verdickung wasserbasierter Dispersionsfarben eingesetzt.

Als Wirkprinzip für die verdickende Wirkung der HEUR-Verdicker wird angenommen, dass die Polyethylenglykolsegmente die Wasserverträglichkeit sicherstellen und die Hydrophobsegmente über eine Assoziation untereinander sowie mit dispergierten Bindemittelteilchen der zu verdickenden Dispersionsfarbe in dieser einen viskositätsgebenden dreidimensionalen Molekülverbund aufbauen.

Die Verwendung der bekannten Verdickungsmittel ist jedoch, je nach der zu verdickenden Zubereitung, mit Nachteilen verbunden. So kann die Verdickungswirkung und die Salzstabilität der Verdickungsmittel nicht zufrieden stellend und ihre Einarbeitung in die zu verdickende Zubereitung erschwert sein. Es ist beispielsweise bekannt, dass viele Verdicker wie z.B. vernetzte (hydrophob modifizierte) Polyacrylsäuren in neutralisiertem Zustand oder auch polyurethanhaltige Verdicker sehr empfindlich auf Salz oder Tensid oder eine Mischung derselben reagieren. Salzzugabe kann zu abrupter und drastischer Viskositätserniedrigung führen. Deshalb ist es beispielsweise unüblich, diese Polymere in Shampoo-Formulierungen als Viskositätsgeber einzusetzen. Aufgrund der dort vorliegenden Salzkonzentrationen (Tenside, Tensid-Gemische, NaCl als Verunreinigung in Tensiden) kann keine signifikante Viskositätserhöhung herbeigeführt werden. Bei Anwesenheit von kationischen Hilfsstoffen kann es zur Komplexbildung und Niederschlag kommen. Auch im Bereich der kosmetischen Zubereitungen werden Verdicker eingesetzt. Allerdings sind für kosmetische Zubereitungen derzeit keine salzstabilen Verdicker bekannt, die bei guter Verdickungsleistung in Gegenwart von Salz auch zu Zubereitungen mit guter Textur und angenehmen Gefühl auf der Haut führen. Die Verträglichkeit mit zahlreichen anderen Hilfsstoffen, insbesondere mit Salzen und Tensiden und auch die Einarbeitbarkeit des Verdickers selbst sowie der weiteren Hilfsstoffe sollte gegeben sein. Außerdem müssen die verdickten Zubereitungen auch bei Langzeitlagerung, Temperatur- und pH-Veränderungen eine gleichbleibende Rheologie und physikalische und chemische Qualität aufweisen. Letztlich sollen diese Verdicker noch kostengünstig und ohne eine merkliche Umweltbelastung herzustellen sein.

Die Schriften US 4 079 028 und US 4 155 892 offenbaren unter anderem lineare Polyurethan-Verdicker. Die Herstellung dieser Polyurethan-Verdicker erfolgt in Gegenwart von Polymerisationskatalysatoren.

EP 1 584 331-A und EP 1 013 264 B offenbaren Polyurethan-Verdicker für kosmetische Zubereitungen. Diese werden in einem Ein-Schritt-Verfahren durch Reaktion in Substanz aus Polyol, Polyisocyanat und Fettalkohol, der gewünschtenfalls ethoxyliert sein kann, hergestellt. Die Viskosität einer Zubereitung, die diese Verdicker enthalten, ändert sich laut der genannten Schriften nicht, wenn sich die Salzkonzentration in der Zubereitung ändert.

WO 2006/1 002 813 A offenbart Polyurethan-Verdicker für verschiedene Anwendungen in wässrigen Medien. Diese Verdicker werden aus hydrophilen Polyolen mit mindestens zwei Hydroxygruppen, ein oder mehr hydrophoben Verbindungen z.B. langkettigen Alkoholen und mindestens difunktionalen Isocyanaten hergestellt. Dabei wird ein Überschuss an NCO-Gruppen eingesetzt. Der bei der Herstellung eingesetzte Katalysator kann zinnhaltig, zinkhaltig oder ein Amin sein.

EP 0 725 097 B offenbart Polyurethan-Verdicker, bei deren Herstellung Polyether, erzeugt durch Alkoxylierung von Alkoholen oder Alkylphenolen, mit Polyisocyanaten umgesetzt werden, wobei das Verhältnis von NCO zu OH-Äquivalenten im Bereich von 0,9:1 bis 1,2:1 liegt. Diese Verdicker werden für den Einsatz im Bereich niedriger Scherkräfte z.B. für den Verlauf von wässrigen Dispersionsfarben vorgeschlagen.

Eine Aufgabe der vorliegenden Erfindung war es, neue Polyurethane zur Verfügung zu stellen, die in Wasser dispergierbar sein sollten. Eine weitere Aufgabe der vorliegenden Erfindung war es, neue Verdicker für Wasser enthaltende Zubereitungen zur Verfügung zu stellen. Noch eine weitere Aufgabe der vorliegenden Erfindung war es, neue Verdicker für Wasser enthaltende Zubereitungen, beispielsweise für kosmetische Zubereitungen, zur Verfügung zu stellen, welche zu möglichst hohen Viskositäten führen. Darüber hinaus sollten Verdicker gefunden werden, die in Gegenwart von Salz stabile oder sogar gesteigerte Viskositäten in Wasser enthaltenden Zubereitungen erzeugen. Ziel war des Weiteren die Bereitstellung von Polyurethanverdickern mit den beschriebenen Eigenschaften, die zusätzlich zinnfrei sind, da dies für kosmetische Anwendungen erwünscht ist. Eine sich weiter stellende Aufgabe war ein Verfahren zur Herstellung von in Wasser dispergierbaren Polyurethanen zur Verfügung zu stellen, bei dem möglichst einheitlich strukturierte Moleküle entstehen und ungewollte Nebenprodukte oder ungewollte Vernetzungsreaktionen vermindert sind, obwohl das Verfahren ohne die in der Polyurethanchemie gängigen Zinn-haltigen Katalysatoren arbeitet. Ferner sollte für in Wasser dispergierbare Polyurethane ein Herstellungsverfahren bereitgestellt werden, bei dem ausschließlich aliphatische Isocyanat-Komponenten eingesetzt werden. Des Weiteren sollte für in Wasser dispergierbare Polyurethane ein Herstellungsverfahren bereitgestellt werden, bei dem möglichst geringe Mengen von Isocyanaten eingesetzt werden.

Die in Wasser dispergierbaren Polyurethane PU der vorliegenden Erfindung, die erfindungsgemäßen Verfahren zu deren Herstellung, die erfindungsgemäße Verwendung derselben Polyurethane in Wasser enthaltenden Zubereitungen und Wasser enthaltenden Zubereitungen, die die erfindungsgemäße Polyurethane enthalten, stellen Lösungen der oben beschriebenen Aufgaben dar.

Erfindungsgemäß sind die Polyurethane in Wasser dispergierbar. Erfindungsgemäß umfasst dies, dass sie sich auch in Wasser emulgieren oder in Wasser vollständig oder teilweise lösen können.

Bevorzugt weisen die erfindungsgemäßen Polyurethane PU die Eigenschaft auf, dass sie in einer Dispersion in Wasser bei Konzentrationen zwischen 0,1 g/L und 10 g/L Mizellen mit einer mittleren Teilchengröße kleiner gleich 200 nm ausbilden, insbesondere kleiner gleich 100 nm (bestimmbar mittels dynamischer Lichtstreuung wie nachfolgend beschrieben). Daher kann auch von nanodispergierbaren Polyurethanen gesprochen werden. Die kritische Stoffkonzentration zur Mizellenbildung, auch kritische Mizellenkonzentration (im Englischen "Critical Micelle Concentration" CMC) ist demzufolge bevorzugt kleiner als 0,1 g/L.

Die erfindungsgemäßen Polyurethane weisen ein im Wesentlichen lineares Rückgrat auf, d.h. sie haben keine oder im Verhältnis zur Gesamtlänge wenige Verzweigungsstellen. Verzweigungen davon können in hydrophoben und/oder hydrophilen Abschnitten enthalten sein. Die erfindungsgemäßen Polyurethane PU sind aber nicht sternförmige oder vernetzte Polyurethane. Derartige Polyurethane und deren Herstellung sind aus dem Stand der Technik bekannt und nicht Teil dieser Erfindung.

Bevorzugt besitzen die erfindungsgemäßen Polyurethane pro Molekül kleiner gleich 4 Verzweigungen, besonders bevorzugt kleiner gleich 3 Verzweigungen pro Molekül. In einer besonders bevorzugten Ausführungsform weisen die erfindungsgemäßen Polyurethane außerhalb der randständigen Abschnitte T keine Verzweigungen auf. Dem Fachmann sind Methoden zur Verzweigungsbestimmung wie z.B. über NMR Spektroskopie bekannt.

Das Rückgrat der erfindungsgemäßen Polyurethane ist aufgebaut aus alternierenden hydrophoben und hydrophilen Abschnitten, wobei die hydrophoben und hydrophilen Abschnitte sich zwar in der Abfolge abwechseln, aber in ihrer Größe, Länge und Natur unterschiedlich sein können. Ein hydrophiler Abschnitt schließt sich an beiden Seiten direkt an einen hydrophoben Abschnitt an. Diese hydrophoben Abschnitte können zueinander unabhängig verschieden oder auch identisch sein. Jeder Abschnitt kann kurzkettig oder ein Oligomerrest oder ein Polymerrest sein.

Hydrophil bezeichnet dabei solche Abschnitte, die eine ausgeprägte Wechselwirkung mit Wasser zeigen. Im Allgemeinen bestehen hydrophile Abschnitte aus Resten von Stoffen, die selbst hydrophil sind.

Dem Fachmann bekannte typische hydrophile Gruppen sind nichtionische Polyether-Reste. Bevorzugte Polyether-Reste enthalten im Wesentlichen unverzweigte Alkylenoxidreste.

Polyether-Reste können homo-Alkylenoxidreste sein, oder Mischungen unterschiedlicher Alkylenoxidreste enthalten. Diese unterschiedlichen Alkylenoxidreste können statistisch verteilt in den Polyether-Resten vorhanden sein oder blockförmig vorliegen.

Bevorzugte Polyether-Reste sind homo-Ethylenoxidreste oder homo-Propylenoxidreste. Nach einer anderen Ausführungsform enthalten die Polyether-Reste Mischungen aus Ethylenoxidresten und Propylenoxidresten. Diese können statistisch verteilt in den Polyether-Resten vorhanden sein oder blockförmig vorliegen.

Zu einer besonders bevorzugten Ausführungsform gehören Polyether-Reste, die mindestens 50 Gew.-% Ethylenoxidreste aufweisen, beispielsweise Polyether-Reste, die über 50 Gew.-% Ethylenoxidreste und als weitere Alkylenoxidreste Propylenoxidreste aufweisen. Ganz besonders bevorzugt bestehen die Polyether-Reste aus Ethylenoxidresten.

Die Hydrophilie eines Stoffes kann beispielsweise durch eine Trübungsmessung einer wässrigen Lösung bestimmt werden.

Die in den erfindungsgemäßen Polyurethanen enthaltenen hydrophoben Abschnitte verhalten sich gegenüber Wasser entgegengesetzt den hydrophilen Abschnitten. Im Allgemeinen bestehen die hydrophoben Abschnitte aus Resten von Substanzen die sich nicht oder nur sehr schlecht mit Wasser mischen und so gut wie immer lipophil sind, das heißt sie lösen sich gut in unpolaren Lösungsmitteln, Fetten und Ölen. Typische hydrophobe Gruppen sind beispielsweise Kohlenwasserstoffreste, insbesondere langkettige Kohlenwasserstoffreste. Erfindungsgemäß sind unverzweigte oder gering verzweigte Kohlenwasserstoffreste bevorzugt. Nach einer der Ausführungsformen sind die Kohlenwasserstoffreste unverzweigt. Langkettige aliphatische Alkohole, aromatische Alkohole sowie aliphatische Diisocyanate sind Beispiele für hydrophobe Substanzen, deren Reste in den hydrophoben Abschnitten der erfindungsgemäßen Polyurethane enthalten sein können.

Ein Molekül, das sowohl hydrophobe wie auch hydrophile Abschnitte aufweist, wird im Allgemeinen als amphiphiles Molekül bezeichnet. Als Beispiele sind u.a. Phospholipide, Emulgatoren und Tenside zu nennen. Ein Maß für die Hydrophilie einer amphiphilen Verbindung stellt der HLB Wert dar. Der HLB-Wert (englische Abkürzung für: hydrophilic-lipophilic-balance) beschreibt den hydrophilen und lipophilen Anteil von hauptsächlich nichtionischen Tensiden und wurde im 20. Jahrhundert von W. C. Griffin vorgeschlagen (Griffin, W. C.: Classification of surface active agents by HLB, J. Soc. Cosmet. Chem. 1, 1949).

Der HLB-Wert kann folgendermaßen berechnet werden (siehe Formel I):

$$HLB = 20 * \left( 1 - \frac{M_l}{M} \right) \quad \text{(Formel I)}$$

wobei $M_l$ die Molmasse des hydrophoben Anteils eines Moleküls ist und M die Molmasse des gesamten Moleküls. Der Faktor 20 ist ein von Griffin frei ausgewählter Skalierungsfaktor. Es ergibt sich damit in der Regel eine Skala von 1 bis 20. Ein HLB-Wert von 1 spricht für eine lipophile Verbindung, eine chemische Verbindung mit einem HLB-Wert von 20 hat einen hohen hydrophilen Anteil.

Die erfindungsgemäßen Polyurethane haben bevorzugt einen HLB Wert nach Griffin von größer gleich 10, besonders bevorzugt von größer gleich 14, auf einer Skala von 1 bis 20.

Erfindungsgemäße Polyurethane enthalten mindestens zwei endständige hydrophobe Abschnitte (T). Die erfindungsgemäßen Polyurethane PU können in geringem Maße im Molekülinnern verzweigt sein (gewünschtenfalls durch Verwendung von Tri-oder Polyisocyanaten in geringen Anteilen), so dass dann mehr als zwei endständige hydrophobe Abschnitte T enthalten sein könnten. Bevorzugt sind die erfindungsgemäßen Polyurethane PU im Molekülinnern unverzweigt und enthalten zwei endständige hydrophobe Abschnitte T. Ihre Endständigkeit bedingt, dass sie sich nur an einen weiteren Abschnitt der erfindungsgemäßen Polyurethane direkt anschließen.

Die endständigen Abschnitte T können gleich oder unabhängig voneinander verschieden sein.

Die endständigen hydrophoben Abschnitte T können verzweigt oder unverzweigt sein. Bevorzugt ist mindestens einer der beiden endständigen hydrophoben Abschnitte T der erfindungsgemäßen Polyurethane PU verzweigt.

Bevorzugt enthalten die endständigen hydrophoben Abschnitte T eine Kette aus Kohlenstoffatomen. Bevorzugt liegt die Kettenlänge der Abschnitte T im Bereich von 4 bis 30 Kohlenstoffatomen, besonders bevorzugt im Bereich von 6 bis 26 und ganz besonders bevorzugt im Bereich von 8 bis 20 Kohlenstoffatomen.

Derartige Abschnitte T können beispielsweise aus aromatischen Resten, aber auch aus Alkylresten bestehen. So können die Abschnitte T verzweigte oder unverzweigte Alkylreste sein, oder diese enthalten. Bevorzugt ist mindestens ein Abschnitt T ein verzweigter Alkylrest. Verzweigt bedeutet, dass an einem oder auch mehreren Kohlenstoffatomen des Alkylrests Verzweigungen ansetzen. Üblicherweise bedeutet eine Verzweigung eines Alkyls, dass neben den Mitgliedern der Hauptkette ein oder mehrere zusätzliche Kohlenstoffatome an ein oder zwei Stellen an einem Kohlenstoffatom des Kohlenstoffrückgrats kovalent gebunden sind, und eine Seitenkette bilden. Die Seitenketten können identische oder unterschiedliche Größen haben. Bevorzugt sind die Seitenketten selbst Alkylreste oder Alkylenreste, besonders bevorzugt Alkylreste, insbesondere unverzweigte Alkylreste.

In einer Ausführungsform weisen die Seitenketten der Alkylreste bevorzugt eine Kettenlänge von nicht mehr als 6 Kohlenstoffatomen auf. In einer anderen Ausführungsform sind die Verzweigungen vorzugsweise deutlich kürzere Ketten als die Hauptkette. Vorzugsweise hat jede Verzweigung der Abschnitte T der erfindungsgemäßen Polyurethane höchstens eine Kettenlänge, die der Hälfte der Kettenlänge der Hauptkette dieses Abschnitts T entspricht. Besonders bevorzugt sind die verzweigten Alkylreste iso- und/oder neo-Alkylreste. Bevorzugt liegt die Kettenlänge der Hauptkette von Alkylresten, die in Abschnitten T enthalten sind, im Bereich von 4 bis 30 Kohlenstoffatomen, beispielsweise Alkylreste von Butan, Pentan, Hexan, Heptan, Octan, Nonan, Decan, Undecan, Dodecan, Tridecan, Tetradecan, Pentadecan, Hexadecan, Heptadecan, Octadecan, Nonadecan, Icosan, Henicosan, Docosan, Tricosan, Tetracosan, Pentacosan, Hexacosan, Heptacosan, Octacosan, Nonacosan und/oder Triacontan. Verzweigte Alkyreste dieser Alkane können verwendet werden. Ebenso können auch Reste von Cylcoalkanen oder Alkenen enthalten sein. Besonders bevorzugt enthalten die Abschnitte T Alkylreste mit einer Anzahl von Kohlenstoffatomen im Bereich von 6 bis 26, beispielsweise Reste von Hexan, Heptan, Octan, Nonan, Decan, Undecan, Dodecan, Tridecan, Tetradecan, Pentadecan, Hexadecan, Heptadecan, Octadecan, Nonadecan, Icosan, Henicosan, Docosan, Tricosan, Tetracosan, Pentacosan und/oder Hexacosan, und ganz besonders bevorzugt im Bereich von 8 bis 20 Kohlenstoffatomen, beispielsweise Reste von Octan, Nonan, Decan, Undecan, Dodecan, Tridecan, Tetradecan, Pentadecan, Hexadecan, Heptadecan, Octadecan, Nonadecan und/oder Icosan. Verzweigte Alkylreste dieser Alkane können genauso verwendet werden wie auch Reste von Cylcoalkanen oder Alkenen.

In einer bevorzugten Ausführungsform werden als verzweigte Alkylreste Reste von Iso-Alkanen verwendet. Besonders bevorzugt ist ein C13-Alkylrest, insbesondere ein iso-C13 Alkylrest.

Die Einführung der Abschnitte T in die erfindungsgemäßen Polyurethane kann auf verschiedene Weise erfolgen, beispielsweise als Teil von ethoxylierten Fettalkoholen.

Erfindungsgemäß sind auch Mischungen von Polyurethanen PU, deren endständige, hydrophobe Abschnitte T verzweigte und/oder unverzweigte Alkylreste sind. Beispielsweise auch Mischungen, in denen erfindungsgemäße Polyurethane PU enthalten sind, die sowohl verzweigte als auch unverzweigte endständige, hydrophobe Abschnitte T haben.

Direkt anschließend an jeden Abschnitt T befindet sich in erfindungsgemäßen Polyurethanen ein hydrophiler Abschnitt (S). Der Abschnitt S wirkt abstandsgebend als so genannter Spacer S. Eine gewisse räumliche Flexibilität der Abschnitte S ist gewünscht. Bevorzugt sind die hydrophilen Abschnitte unverzweigt.

In den erfindungsgemäßen Polyurethanen PU können die Spacer S gleich oder unabhängig voneinander verschieden sein. In einer Ausführungsform sind die hydrophilen Abschnitte S von verschiedener Länge und linear.

In einer weiteren bevorzugten Ausführungsform haben die Abschnitte S eine Kettenlänge von 5 bis 100 Atomen, bevorzugt von 6 bis 90 Atomen und besonders von 8 bis 80 Atomen, insbesondere Ketten von 15 bis 60 Atomen.

Die Abschnitte S können Reste von Alkylenoxiden enthalten. Bevorzugt liegt die Anzahl im Bereich von 2 bis 30 Alkylenoxidresten, besonders bevorzugt im Bereich von 3 bis 25 Alkylenoxidresten und ganz besonders bevorzugt im Bereich von 3 bis 20 Alkylenoxidresten.

Erfindungsgemäß bevorzugt bestehen die mindestens zwei hydrophilen Abschnitte S Ethylenoxidresten. Die hydrophile Abschnitte S enthalten in einer bevorzugten Ausführungsform Ethylenoxidreste, deren Anzahl im Bereich von 2 bis 30 Resten liegt, besonders bevorzugt im Bereich von 3 bis 25 Ethylenoxidresten und ganz besonders bevorzugt im Bereich von 3 bis 20 Resten.

Auch eine Mischung aus Ethylenoxid- und Propylenoxidresten oder nur Propylenoxidreste in Anschnitten S sind möglich. Ebenso können Abschnitte S längerkettige Alkylenoxidreste enthalten, wobei allerdings beachtet werden muss, dass die Abschnitte S insgesamt hydrophil sein müssen (z.B. durch entsprechend hohen Ethylenoxid-Anteil).

An jeden hydrophilen Abschnitt S schließt sich direkt auf mindestens einer Seite mindestens ein hydrophober Abschnitt (D) an. Dabei kann ein Abschnitt S auch im Molekülinnern der erfindungsgemäßen Polyurethane enthalten sein. In diesem Fall ist dieser Abschnitt S nicht wie ein randständiger Abschnitt S mit einem Abschnitt D und einem Abschnitt T, sondern auf mindestens zwei Seiten mit Abschnitten D verbunden. Bevorzugt ist ein Abschnitt S im Molekülinnern auf beiden Seiten mit je einem Abschnitt D verbunden. Für alle randständigen Abschnitte S gilt, dass sie mit einem endständigen Abschnitt T direkt verbunden sind.

Sollte ein Abschnitt S in geringem Maße verzweigt sein, so könnte er an zwei oder mehr Stellen mit hydrophoben Abschnitten D direkt verbunden sein. Bevorzugt schließen sich an jeden linearen hydrophilen Spacer S auf ein oder zwei Seiten je ein hydrophober Abschnitt D an.

In einer besonders bevorzugten Ausführungsform sind alle, d.h. insbesondere die zwei Abschnitte S unverzweigt, randständig, und mit einem Abschnitt T auf der einen Seite und einem Abschnitt D auf der anderen Seite verbunden.

Erfindungsgemäß enthalten die Polyurethane mindestens zwei hydrophobe Abschnitte D. Die hydrophoben Abschnitte D können gleich oder unabhängig voneinander verschieden sein.

Die Abschnitte D können verzweigt mit kurzkettigen hydrophoben Verzweigungen oder unverzweigt sein. Bevorzugt sind die Abschnitte D unverzweigt.

Bevorzugt enthalten die Abschnitte D eine hydrophobe Kette von Kohlenstoffatomen, deren Länge im Bereich von 2 bis 20 Kohlenstoffatomen, bevorzugt 3 bis 16 Kohlenstoffatomen und insbesondere im Bereich von 4 bis 12 Kohlenstoffatomen liegt.

Bevorzugt enthalten die Abschnitte D Diisocyanatreste. Besonders bevorzugt enthalten die Abschnitte D Reste von aliphatischen Diisocyanaten. So kann beispielsweise ein hydrophober Abschnitt D aus einem oder mehreren aliphatischen Diisocyanatresten bestehen. Bevorzugt besteht ein Abschnitt D aus einem bis zehn aliphatischen Diisocyanatresten, besonders bevorzugt aus einem bis zu fünf aliphatischen Diisocyanatresten, ganz besonders bevorzugt enthält er ein, zwei oder drei aliphatische Diisocyanatreste.

Die hydrophoben Abschnitte D können aliphatische Diisocyanatreste mit langen, mittellangen oder kurzen aliphatischen Einheiten enthalten.

In einer der bevorzugten Ausführungsformen handelt es sich bei den Abschnitten D der erfindungsgemäßen Polyurethane um cycloaliphatische oder aliphatische Diisocyanatreste. Besonders bevorzugt sind die Abschnitte D aliphatische Diisocyanatreste.

Als aliphatische Diisocyanate seien beispielhaft genannt: 1,4-Butylendiisocyanat, 1,12-Dodecamethylendiisocyanat, 1,10-Decamethylendiisocyanat, 2-Butyl-2-ethylpentamethylendiisocyanat, 2,4,4- oder 2,2,4-Trimethylhexamethylendiisocyanat und insbesondere Hexamethylendiisocyanat (HDI).

Als cycloaliphatische Diisocyanate seien beispielhaft genannt: Isophorondüsocyanat (IPDI), 2-Isocyanatopropylcyclohexylisocyanat, 4-Methyl-cyclohexan-1,3-diisocyanat (H-TDI) und 1,3-Bis-(isocyanatomethyl)-cyclohexan. Auch sogenannte $H_{12}$-MDI oder im Englischen "saturated MDI" genannte Diisocyanate, wie z.B. 4,4'-Methylen-bis-(cyclohexylisocyanat) (alternativ auch Dicyclohexylmethan-4,4'-diisocyanat genannt) oder 2,4'-Methylenbis(cyclohexyl)-diisocyanat können als Reste in Abschnitten D der erfindungsgemäßen Polyurethane PU enthalten sein.

Selbstverständlich kann man Mischungen der vorstehend genannten Diisocyanate einsetzen, um Mischungen unterschiedlicher erfindungsgemäßer Polyurethane PU herzustellen.

Die erfindungsgemäßen Polyurethane enthalten mindestens einen hydrophilen Abschnitt (P). Dabei gilt, dass mindestens ein hydrophober Abschnitt D sich an P an mindestens einer Seite direkt anschließt. Die Abschnitte P der erfindungsgemäßen Polyurethane können gleich oder unabhängig voneinander verschieden sein.

Ist in einem erfindungsgemäßen Polyurethan mehr als ein Abschnitt P enthalten, so befindet sich zwischen den hydrophilen Abschnitten P mindestens ein hydrophober Abschnitt D. Erfindungsgemäße Polyurethane können in einer Ausführungsform zwischen zwei hydrophilen Abschnitten P eine Sequenz von Abschnitten der Reihenfolge hydrophober Abschnitt D, dann hydrophiler Abschnitt S, dann wieder hydrophober Abschnitt D enthalten. Sind in einem erfindungsgemäßen Polyurethan also mehr als ein Abschnitt P enthalten, so können in einem solchen Fall die Abschnitte im Molekülinnern eine Abfolge von P-D-P oder von P-D-S-D-P aufweisen. Sollten mehr als zwei Abschnitte P enthalten sein, so sind beide Abfolgen in einem Molekül möglich. Bevorzugt sind in einem Molekül der erfindungsgemäßen Polyurethane nur ein oder zwei Abschnitte P enthalten.

Bevorzugt sind die hydrophilen Abschnitte P im Wesentlichen lineare Polyetherreste, z.B. Polyalkylenoxide. Besonders bevorzugt sind die hydrophilen Abschnitte P Reste von Polyetherdiolen, insbesondere von Polyethylenglykolen. Der mindestens eine hydrophile Abschnitt P der erfindungsgemäßen Polyurethane ist bevorzugt aus Polyethylenoxid aufgebaut.

Erfindungsgemäß haben die im Wesentlichen linearen Polyetherreste, die die Abschnitte P bilden, ein zahlenmittleres Molekulargewicht von mindestens 1500 g/mol aufzuweisen. Im Allgemeinen weisen die Abschnitte P Molekulargewichte mittlerer Größe auf, z.B. bis zu 20 000 g/mol.

In einer besonders bevorzugten Ausführungsform haben die im Wesentlichen linearen Polyetherreste zahlenmittlere Molekulargewichte im Bereich von 1500 g/mol bis 12000 g/mol. Besonders bevorzugt ist das Molekulargewicht der Abschnitte P kleiner oder gleich 10000 g/mol und insbesondere bevorzugt im Bereich von 4000 g/mol bis 9000 g/mol. Ganz besonders bevorzugt haben die linearen Polyetherreste Molekulargewichte von größer oder gleich 6000 g/mol.

Sämtliche hydrophile Abschnitte der erfindungsgemäßen Polyurethane, d.h. sowohl Abschnitte S wie auch Abschnitte P, können Polyetherreste sein.

In einer bevorzugten Ausführungsform bestehen die hydrophilen Abschnitte der erfindungsgemäßen Polyurethane aus

- Polyalkylenoxid-Einheiten (Abschnitte P) und
- Polyethylenoxid-Einheiten (Abschnitte S).

In einer besonders bevorzugten Ausführungsform der erfindungsgemäßen PU bestehen alle Abschnitte P und S aus Polyethylenoxid-Einheiten.

Das Rückgrat der erfindungsgemäßen Polyurethane enthält im Wesentlichen Reste von Polyethern und Diisocyanaten.

Die erfindungsgemäßen Polyurethane enthalten mindestens drei hydrophile Abschnitte. In einer der bevorzugten Ausführungsformen sind dies zwei Abschnitte S und mindestens ein Abschnitt P.

In einer besonders bevorzugten Ausführungsform ist die Abfolge der Abschnitte der erfindungsgemäßen Polyurethane entweder T-S-D-P-D-S-T oder T-S-D-P-D-P-D-S-T.

Für jeden Abschnitt P gilt, dass seine Größe im Vergleich zu der Größe jedes im gleichen Molekül enthaltenen Spacers S größer ist.

Das Verhältnis der Molekulargewichte eines jeden hydrophilen Abschnittes S der erfindungsgemäßen Polyurethane zu dem Molekulargewicht eines jeden hydrophilen Abschnittes P liegt im Bereich von 1 zu 1,4 bis 1 zu 140, bevorzugt im Bereich von 1 zu 1,7 bis 1 zu 120. In einer bevorzugten Ausführungsform ist das Verhältnis gleich 1 zu x, wobei x gleich oder größer 2 ist, bevorzugt gleich oder größer 2,3 und besonders bevorzugt ist x gleich oder größer 2,8. Besonders bevorzugt liegt das Verhältnis im Bereich von 1 zu 2,8 bis 1 zu 115, ganz besonders bevorzugt im Bereich von 1 zu 3 bis 1 zu 95 und insbesondere bevorzugt im Bereich von 1 zu 3,4 bis 1 zu 80.

Ebenfalls erfindungsgemäß sind Polyurethane PU wie oben beschrieben, für die zusätzlich gilt, dass sie eine Mischung darstellen. Eine derartige Mischung kann z.B. Polyurethane enthalten, die zwar die gleiche Abfolge der Abschnitte T, S, D und / oder P aufweisen, wobei sie sich aber in mindestens einem der Abschnitte strukturell voneinander unterscheiden. Als Beispiel hierfür sei ein unterschiedlicher Abschnittsaufbau oder eine unterschiedliche Abschnittskettenlänge genannt. So können in einer Mischung erfindungsgemäßer Polyurethane PU Abschnitte T verschieden sein. Beispielsweise kann eine erfindungsgemäße Mischung Polyurethane enthalten, deren Abschnitte T beide verzweigt sind, und/oder solche, deren Abschnitte T beide linear sind, und/oder solche Polyurethane, die einen linearen Abschnitt T und einen verzweigten Abschnitt T enthalten. Selbstverständlich können solche Mischungen auch andere Stoffe wie z.B. weitere, nicht-erfindungsgemäße, bevorzugt wasserdispergierbare Polyurethane enthalten.

Eine solche Mischung von Polyurethanen PU kann durch den Einsatz entsprechend verschiedener Einsatzstoffe bzw. deren Mischungen bei der Herstellung der erfindungsgemäßen Polyurethane PU, erfolgen, oder durch nachträgliche Mischung nur einheitlich hergestellter erfindungsgemäßer Polyurethane erzeugt werden.

In einer Ausführungsform ist die Summe der Molekulargewichte aller Abschnitte T, plus der Molekulargewichte von Abschnitten D kleiner oder gleich der Summe der Molekulargewichte aller Abschnitte P zu halten.

Gegenstand der Erfindung ist außerdem ein Verfahren zur Herstellung von erfindungsgemäßen Polyurethanen PU.

Man kann die erfindungsgemäßen Polyurethane PU in Abwesenheit oder vorzugsweise in Anwesenheit mindestens eines Katalysators herstellen.

Als Katalysatoren kommen beispielsweise alle in der Polyurethanchemie üblicherweise verwendeten Katalysatoren in Betracht.

Besonders bevorzugt verwendet man solche Katalysatoren, welche in organischen Lösungsmitteln wie Xylol, Toluol, Aceton, Tetrahydrofuran (THF), Butylacetat, N-Methylpyrrolidon und/oder N-Ethylpyrrolidon löslich sind.

Üblicherweise in der Polyurethanchemie verwendete Katalysatoren sind organische Amine, insbesondere tertiäre aliphatische, cycloaliphatische oder aromatische Amine, und Lewis-saure organische Metallverbindungen.

Als Lewis-saure organische Metallverbindungen kommen z.B. Metallkomplexe wie Acetylacetonate des Eisens, Titans, Zinks, Aluminiums, Cobalts, Mangans, Nickels und Zirkons in Betracht wie z.B. Zirkon-2,2,6,6-tetramethyl-3,5-heptandionat. Weitere geeignete Metallverbindungen werden von Blank et al. in Progress in Organic Coatings, 1999, 35, 19 ff. beschrieben.

Auch Wismut-, Cobalt- oder Zinkkatalysatoren sowie Cäsium- oder Titansalze können als Katalysatoren eingesetzt werden.

Bevorzugt erfolgt die Herstellung von erfindungsgemäßen Polyurethanen PU in Gegenwart von Zink- und/oder Titan haltigen Verbindungen. Besonders bevorzugt ist die Gegenwart von mindestens einem Zink-Carboxylat oder mindestens einem Titan (IV)-alkoholat oder Mischungen derselben bei der Herstellung der erfindungsgemäßen Polyurethane PU.

In einer Ausführungsform der Erfindung werden Titanalkoholate, bevorzugt mit einer Kettenlänge von 2 oder mehr Kohlenstoffatomen, eingesetzt. In einer bevorzugten Ausführungsform weisen die Titanalkoholate eine Kohlenstoffkette von 20 oder weniger Kohlenstoffatomen auf. Bevorzugt liegt die Kettenlänge der Titanalkoholate im Bereich von 3 bis 18 Kohlenstoffatomen. Besonders bevorzugt sind Titanalkoholate basierend auf aliphatischen Alkoholen. In einer insbesonders bevorzugten Ausführungsform erfolgt die Herstellung der erfindungsgemäßen Polyurethane PU in Gegenwart von Orthotitansäuretetrabutylester, auch als Titan (IV)-butylat oder Tetrabutoxytitan bekannt.

In einer bevorzugten Ausführungsform der Erfindung werden Zink-Carboxylate als Katalysatoren eingesetzt, die in Aceton, Toluol, Xylol und/oder aliphatischen Kohlenwasserstoffen löslich sind.

In einer weiteren bevorzugten Ausführungsform erfolgt die Herstellung der erfindungsgemäßen Polyurethane PU in Gegenwart von mindestens einem Zink-Carboxylat, bei denen das Anion den Formeln $(C_nH_{2n-1}O_2)^-$ oder $(C_{n+1}H_{2n-2}O_4)^{2-}$ mit n gleich 1 bis 20 gehorcht. Besonders bevorzugte Zinksalze weisen als Anionen Monocarboxylate der allgemeinen Formel $(C_nH_{2n-1}O_2)^-$ auf, wobei n für die Zahlen 1 bis 20 steht.

Bevorzugt werden die erfindungsgemäßen Polyurethane PU hergestellt in Gegenwart von Zink-Carboxylaten, die aliphatische oder aromatische Carboxylate sind, und gewünschtenfalls ein oder zwei Ringstrukturen enthalten können.

In einer besonders bevorzugten Ausführungsform werden als Katalysatoren für die Herstellung erfindungsgemäßer Polyurethane PU Zink-Carboxylate bevorzugt, deren Carbonsäurereste eine Kohlenstoffkette von 20 oder weniger, bevorzugt 18, besonders bevorzugt kleiner gleich 12 oder weniger Kohlenstoffatomen aufweisen, da gefunden wurde, dass bei langkettigen Carboxylatresten die Aktivität des Katalysators im erfindungsgemäßen Verfahren abnimmt.

In einer Ausführungsform können Zink-Carboxylate ohne Ringstruktur als Katalysatoren zur Herstellung erfindungsgemäßer Polyurethane verwendet werden. Besonders bevorzugt werden aliphatische Zink-Carboxylate als Katalysatoren verwendet.

Ganz besonders bevorzugt werden als Katalysatoren zum Einsatz in erfindungsgemäßen Verfahren zur Herstellung von erfindungsgemäßen Polyurethanen PU Zink-2-Ethylhexanoat (auch Zink-Octanoat genannt), Zink-n-Octanoat, Zink-n-Decanoat, Zink-Neodecanoat, Zink-Ricinoleat und Zink-Stearat. Insbesondere bevorzugt wird Zink-Neodecanoat verwendet.

Selbstverständlich können auch Gemische aus zwei oder mehreren der vorstehend genannten Verbindungen als Katalysatoren zur Herstellung von erfindungsgemäßen Polyurethanen PU eingesetzt werden.
Bevorzugt wird ein Katalysator verwendet.

Die Menge des eingesetzten Katalysators spielt an sich keine Rolle. Im Allgemeinen wird eine wirtschaftliche Menge an Katalysator verwendet. Daher setzt man den Katalysator oder das Gemisch der Katalysatoren vorzugsweise in einer Menge im Bereich von 100 ppm bis 10 000 ppm bezogen auf das Gesamtgewicht der eingesetzten Polyetherdiole ein. Bevorzugt zur Herstellung der erfindungsgemäßen Polyurethane PU wird Katalysator in einer Menge im Bereich von 500 bis 5000 ppm ein, besonders bevorzugt in einer Menge gleich oder kleiner 4500 ppm bezogen auf das Gesamtgewicht der eingesetzten Polyetherdiole. In einer besonders bevorzugten Ausführungsform wird eine Menge Katalysator im Bereich von 1000 ppm bis 3000 ppm bezogen auf das Gesamtgewicht der eingesetzten Polyetherdiole zur Herstellung der erfindungsgemäßen Polyurethane verwendet.

Man kann -je nach Beschaffenheit des Katalysators oder der Katalysatoren - den oder die Katalysatoren in fester oder flüssiger Form oder gelöst in den erfindungsgemäßen Verfahren zusetzen. Geeignete Lösemittel sind mit Wasser nicht mischbare Lösungsmittel wie aromatische oder aliphatische Kohlenwasserstoffe, u.a. Toluol, Xylol, Ethylacetat, Hexan und Cyclohexan, sowie Carbonsäureester wie beispielsweise Ethylacetat. Weiterhin geeignete Lösemittel sind Aceton, THF und N-Methylpyrrolidon und N-Ethylpyrrolidon. Bevorzugt setzt man den oder die Katalysatoren in fester oder flüssiger Form zu. Bevorzugt setzt man den Katalysator gelöst in einem Lösemittel ein, ganz besonders bevorzugt gelöst in organischen Lösungsmitteln wie aliphatischen Kohlenwasserstoffen, Aceton, Toluol oder Xylol.
In einer besonders bevorzugten Ausführungsform der Erfindung werden der oder die Katalysatoren in gelöster Form eingesetzt.
In einer weiteren besonders bevorzugten Ausführungsform der Erfindung werden Zink-Carboxylate als Katalysator eingesetzt, die in aliphatischen Kohlenwasserstoffen, Aceton, Toluol, Xylol bzw. optional deren Gemischen gelöst sind.

Die erfindungsgemäßen Polyurethane PU werden durch ein erfindungsgemäßes Verfahren hergestellt, bei dem die Synthese in zwei Stufen erfolgt. Gewünschtenfalls folgt der zweiten Reaktionsstufe eine Aufarbeitung der Produkte.

Prinzipiell kann die Reaktion auch ohne Katalysator durchgeführt werden, allerdings sind die Produkte im Allgemeinen schlechter reproduzierbar (betrifft z.B. die zahlenmittleren und gewichtsmittleren Molekulargewichte), die Reaktionszeiten sind im Allgemeinen deutlich länger und die erzielten Viskositäten in Zubereitungen, die Wasser enthalten, sind z.T. geringer. In einigen Fällen trat durch die verstärkte Bildung von (hochmolekularen) Nebenprodukten Vernetzung auf, wenn kein Katalysator anwesend war. Bevorzugt wird bei den erfindungsgemäßen Verfahren mindestens ein, besonders bevorzugt genau ein Katalysator eingesetzt.
Ein Vorteil der erfindungsgemäßen Verfahren zur Herstellung von Polyurethanen PU in dieser bevorzugten Ausführungsform ist die Tatsache, dass das Produkt einheitlich strukturierte Moleküle oder eine klar definierte Mischung von Polyurethanmolekülen enthält.

Das erfindungsgemäße Verfahren zur Herstellung von Polyurethanen PU kann in einer Ausführungsform die folgenden Schritte enthalten:

1. Man setzt mindestens ein Polyetherdiol mit einem Molekulargewicht von mindestens 1500 g/mol mit mindestens einem aliphatischen Diisocyanat und in Gegenwart mindestens eines Zink-Carboxylates und/oder mindestens eines Titanalkoholates um;
2. anschließend setzt man die erzeugten Zwischenprodukte mit mindestens einem ethoxylierten Fettalkohol um;
3. danach erfolgt die Aufarbeitung, d.h. in der Regel die Entfernung aller org. Lösungsmittel und die Überführung des Polymers in Wasser.

Die Umsetzung der Einsatzstoffe kann bei den erfindungsgemäßen Verfahren in Lösung erfolgen. Es ist auch eine Reaktion in Schmelze möglich, bei der die Einsatzstoffe nicht oder zum größten Teil nicht gelöst in Lösungsmittel vorliegen.

In einer bevorzugten Ausführungsform des erfindungsgemäßen Verfahrens wird die Umsetzung in zwei Schritten in Lösung durchgeführt, insbesondere bevorzugt gelöst in organischen Lösungsmitteln wie Aceton, Toluol oder Xylol.

Bevorzugt wird möglichst wasserfreies Polyetherdiol im ersten Schritt der erfindungsgemäßen Verfahren eingesetzt. Das Entfernen des Wassers aus dem Polyether kann in erfindungsgemäßen Verfahren durch azeotrope Destillation, Trocknen unter Vakuum oder andere dem Fachmann bekannte Methoden erfolgen. Beispielsweise kann durch azeotrope Destillation Wasser entfernt werden, bis der Wassergehalt vor Zugabe der Diisocyanate bei ungefähr 300 ppm liegt. Die Vorbereitung der eigentlichen Reaktion kann beispielsweise daraus bestehen, dass

entweder das Polyetherdiol unter Vakuum gesetzt und so das Wasser ausreichend (bevorzugt bis zu einem Wassergehalt von ungefähr 300 ppm oder niedriger) entfernt wird, und danach ein Lösungsmittel beigemischt wird, oder das Polyetherdiol mit einem Lösungsmittel wie Xylol, Toluol oder Aceton vermischt wird und das Wasser durch azeotrope Destillation entfernt wird, beispielsweise bis zu einem Wassergehalt von ungefähr 300 ppm, wobei allerdings das Lösungsmittel nicht vollständig entfernt wird, sondern die Lösung von Polyether im verbliebenen Lösungsmittel zur Reaktion in Lösung verwendet wird.

Der pH Wert der Diollösung in Lösungsmittel kann vor der Umsetzung mit Diisocyanaten auf einen Wert von kleiner gleich pH 7 eingestellt und gewünschtenfalls abgepuffert werden, beispielsweise durch Entsalzen oder Zugabe von einer oder einer Mischung unterschiedlicher Säuren. Als Säuren kommen anorganische oder organische Säuren in Betracht, z.B. Salzsäure, Schwefelsäure, Schwefelige Säure, Salpetersäure, Phosphorsäure, Flussäure, Kohlensäure, organische Säuren wie Äpfelsäure, Zitronensäure, Oxalsäure, Ameisensäure, Essigsäure, Propionsäure, Buttersäure.

Die im erfindungsgemäßen Verfahren eingesetzten ethoxylierten Fettalkohole haben vorzugsweise einen Ethoxylierungsgrad, der mindestens im Bereich von 2 bis 30 Resten liegt, besonders bevorzugt im Bereich von 3 bis 25 Ethylenoxidresten und ganz besonders bevorzugt im Bereich von 3 bis 20 Resten. Am meisten bevorzugt wird als mindestens einer der eingesetzten Fettalkohole eine verzweigte, nicht-ionische Verbindung, hergestellt aus einem gesättigtem iso- C13 Alkohol der Strukturformel $RO(CH_2 CH_2O)_x H$, wobei R für einen C13 Alkylrest, bevorzugt einen iso-C13-Alkylrest steht, und mit x = 3,5,6,6.5,7,8,10,12,15 oder 20, bevorzugt x =10 (kommerziell erhältlich von der Firma BASF SE unter der Bezeichnung "Lutensol ® TO" z.B. für x=10 als "Lutensol ® TO10"), verwendet.

Beim erfindungsgemäßen Verfahren kann das Verhältnis (mol zu mol) der eingesetzten Polyetherdiole zu eingesetzten Diisocyanaten im Bereich von 1 zu 1,1 bis 1 zu 1,9 liegen. Bevorzugt liegt das Verhältnis im Bereich von 1 zu 1,1 bis 1 zu 1,8. Besonders bevorzugt liegt das Verhältnis im Bereich von 1 zu 1,1 bis 1 zu 1,75. Insbesondere bevorzugt liegt das Verhältnis im Bereich von 1 zu 1,2 bis 1 zu 1,75. Selbstverständlich kann das Verhältnis auch 1 zu x mit x größer oder gleich 1,3, bevorzugt x größer oder gleich 1,5, sein.

In einer Ausführungsform ergibt sich daraus, dass bevorzugt in einem Molekül der erfindungsgemäßen Polyurethane nur ein oder zwei Abschnitte P enthalten sind.

In einer speziellen Ausführungsform des erfindungsgemäßen Verfahrens (mol zu mol) wird zusätzlich zu den genannten Bereichen des Verhältnisses von Polyetherdiolen zu Diisocyanaten zusätzlich das Verhältnis von Polyetherdiolen zu ethoxylierten Fettalkoholen so gewählt, dass das Verhältnis von eingesetzten Polyetherdiolen zu eingesetzten ethoxylierten Fettalkoholen im Bereich von 5 zu 1 bis 1 zu 2 liegt. Bevorzugt liegt dieses Verhältnis im Bereich von 2 zu 1 bis 1 zu 1,8, besonders bevorzugt im Bereich von 1 zu 1 bis 1 zu 1,6 und am meisten bevorzugt bei 1 zu 1,5. Für alle drei Einsatzstoffe des erfindungsgemäßen Verfahrens gilt, dass ganz besonders bevorzugt ein Verhältnis (mol zu mol) von Polyetherdiolen zu Diisocyanaten zu ethoxylierten Fettalkoholen 1 zu 1,75 zu 1,5 eingesetzt wird.

Gegenstand der Erfindung ist auch die Verwendung der erfindungsgemäßen Polyurethane PU und erfindungsgemäß hergestellter Polyurethane zu Herstellung von Zubereitungen, die Wasser enthalten. Bevorzugt werden dabei Zubereitungen, die mindestens 5 Gew.-%, insbesondere mindestens 20 Gew.-%, ganz besonders bevorzugt mindestens 30 Gew.-% und am meisten bevorzugt mindestens 50 Gew.-% Wasser enthalten. Bei den Wasser enthaltenden Zubereitungen kann es sich beispielsweise um Lösungen, Emulsionen, Suspensionen oder Dispersionen handeln.

Zusätzlich zu den erfindungsgemäßen Polyurethanen PU und erfindungsgemäß hergestellten Polyurethanen können erfindungsgemäß auch andere Stoffe zur Herstellung von Zubereitungen verwendet werden, wie z.B. übliche Hilfsstoffe (beispielsweise Dispergiermittel und /oder Stabilisatoren), Tenside, Konservierungsstoffe, Antischaummittel, Duftstoffe, Netzmittel, Verdicker, Farbstoffe, Weichmacher, Feuchthaltemittel und/oder andere Polymere.

Bevorzugt können die erfindungsgemäßen Polyurethane PU und Mischungen erfindungsgemäß hergestellter Polyurethane verwendet werden, um Wasser enthaltende Zubereitungen herzustellen, die mindestens ein Salz oder mindestens ein Tensid oder Mischungen derselben enthalten.

Unter Tensiden werden im Zusammenhang mit der vorliegenden Erfindung auch Emulgatoren sowie Mischungen aus Tensiden und Emulgatoren verstanden. Unter Salz werden im Zusammenhang mit der vorliegenden Erfindung Salze und auch salzartige Strukturen auch mit niedrigem $pK_S$ Wert und Mischungen derselben verstanden. Besonders bevorzugt werden die erfindungsgemäßen Polyurethane PU und erfindungsgemäß hergestellten Polyurethane verwendet, um Zubereitungen herzustellen, die mindestens 0,05 Gew.-% Salz und/oder mindestens 0,5 Gew.-% Tenside enthalten, ganz besonders bevorzugt mindestens 0,1 % (w/w) Salz und/oder mindestens 1 Gew.-% Tenside enthalten.

In einer weiteren Ausführungsform werden die erfindungsgemäßen Polyurethane PU und erfindungsgemäß hergestellten Polyurethane verwendet, um Zubereitungen herzustellen, die bis 20 Gew.-% Salz, bevorzugt bis 10 Gew.-% und besonders bevorzugt 5 Gew.-% oder weniger Salz enthalten. In einer weiteren Ausführungsform werden die erfindungsgemäßen Polyurethane PU und erfindungsgemäß hergestellten Polyurethane werden verwendet, um Zubereitungen herzustellen, die bis 25 Gew.-% Tenside, bevorzugt bis 20 Gew.-% und besonders bevorzugt 15 Gew.-% oder weniger Tenside enthalten.

In einer weiteren Ausführungsform werden die erfindungsgemäßen Polyurethane PU und erfindungsgemäß hergestellten Polyurethane werden verwendet, um Zubereitungen herzustellen, die bis 10 Gew.-% Salz, bevorzugt bis 5 Gew.-% Salz und bis 20 Gew.-% Tenside, bevorzugt bis 15 Gew.-% Tenside, enthalten.

Besonders bevorzugt werden die erfindungsgemäßen Polyurethane PU und erfindungsgemäß hergestellten Polyurethane verwendet, um Zubereitungen herzustellen, die Öl-in-Wasser-Emulsionen sind. Typischerweise enthalten Öl-in-Wasser-Emulsionen einen Ölanteil größer 0 Gew.-% und kleiner gleich 40 Gew.-%. Bevorzugt werden erfindungsgemäß Öl-in-Wasser-Emulsionen hergestellt, die einen Ölanteil im Bereich von 5 bis 40 Gew.-%, besonders im Bereich von 10 bis 35 Gew.-% und insbesondere von 15 bis 30 Gew.-% Öl enthalten.

Ganz besonders bevorzugt werden die erfindungsgemäßen Polyurethane PU und erfindungsgemäß hergestellten Polyurethane verwendet, um Zubereitungen herzustellen, die Öl-in-Wasser-Emulsionen sind und mindestens ein Salz enthalten.

Bei den erfindungsgemäßen Zubereitungen, die ein erfindungsgemäßes Polyurethan enthalten, kann es sich beispielsweise um Lösungen, Emulsionen, Suspensionen oder Dispersionen handeln.

In einer Ausführungsform ist eine erfindungsgemäße Zubereitung eine Dispersion, bevorzugt eine wässrige Dispersion, der erfindungsgemäßen Polyurethane PU, wie sie nach dem Herstellungsprozess durch Aufarbeitung aus den Reaktionsprodukten erhalten werden kann. Dazu wird beispielsweise das Lösungsmittel entfernt und Wasser zugegeben und eine Dispersion erzeugt. Gewünschtenfalls kann auch die Zugabe eines Konservierungsmittels und/oder Stabilisators erfolgen.

In einer der Ausführungsformen enthält die erfindungsgemäße Dispersion bis zu 25 Gew.-% der erfindungsgemäßen Polyurethane. In einer anderen Ausführungsform enthält die Dispersion 20 Gew.-% Feststoffanteil.

Zusätzlich kann die erfindungsgemäße Dispersion mindestens ein Konservierungsmittel und/oder mindestens einen Stabilisator, der vor Radikalen schützt, enthalten. Ganz besonders bevorzugt sind wässrige Dispersionen enthaltend bis zu 20 % (w/v) der erfindungsgemäßen Polyurethane, einen für kosmetische Anwendungen geeigneten Konservierungsstoff und wahlweise mindestens einen für kosmetische Anwendungen geeigneten Stabilisator, der vor Radikalen schützt. Dem Fachmann sind geeignete Konservierungsstoffe und Radikalstabilisatoren wie z.B. Tocopherol (aber nicht beschränkt auf diesen) bekannt.

Zur Herstellung der erfindungsgemäßen Zubereitungen, die beispielsweise Lösungen, Emulsionen, Suspensionen oder Dispersionen sein können, werden die erfindungsgemäßen Polyurethane bevorzugt in Form wässriger Dispersionen eingesetzt., wie sie aus dem Herstellungsprozess durch Aufarbeitung (beispielsweise durch Entfernen des Lösungsmittels, Zugabe von Wasser und gewünschtenfalls durch Zugabe eines Konservierungsmittels und/oder eines Stabilisators) erhalten werden können.

In erfindungsgemäßen Zubereitungen können in einer weiteren Ausführungsform abhängig vom Einsatzgebiet der Zubereitung weitere Stoffe enthalten sein, wie sie üblicherweise in Zubereitungen verwendet werden. Solche sind, ohne sie erschöpfend aufzuzählen, übliche Hilfsstoffe (beispielsweise Dispergiermittel und /oder Stabilisatoren), Tenside, Konservierungsstoffe, Antischaummittel, Duftstoffe, Netzmittel, Verdicker, Farbstoffe und/oder andere Polymere. Dem Fachmann sind solche weiteren Zusatzstoffe z.B. im Bereich kosmetischer Zubereitungen, Dispersionsfarben oder Zubereitungen von Pflanzenschutzmitteln bekannt.

Bevorzugt ist es erfindungsgemäß, zur Herstellung von Wasser enthaltenden Zubereitungen keine weiteren Verdickungsmittel neben den erfindungsgemäßen Polyurethanen zu verwenden.

Die erfindungsgemäßen Polyurethane PU weisen verschiedene Vorteile auf. Ein Vorteil ist Ihre Eigenschaft, die rheologischen Eigenschaften einer erfindungsgemäßen Zubereitung, die PU enthält, zu verändern.

Unter Modifizierung der rheologischen Eigenschaften wird ganz allgemein die Änderung des Verformungs- und Fließverhaltens von Materie verstanden. Eine der bedeutendsten rheologischen Eigenschaften ist die Viskosität. Dieser Begriff ist dem Fachmann bekannt.

Unter Viskosität versteht man üblicherweise die "Zähigkeit" einer Flüssigkeit. Sie resultiert aus den zwischenmolekularen Kräften in einer Flüssigkeit, ist also abhängig von Kohäsion (intramolekular) und Adhäsion (intermolekular). Die Viskosität charakterisiert das Fließverhalten einer Flüssigkeit. Hohe Viskosität bedeutet Dickflüssigkeit, eine niedrige dagegen Dünnflüssigkeit.

Unter Modifizierung der Rheologie wird insbesondere die Erhöhung der Viskosität von Flüssigkeiten, üblicherweise auch als "Verdickung" bezeichnet, verstanden. Diese Viskositätserhöhung kann bis zur Entstehung von Gelen oder Festkörpern reichen.

Bevorzugt sind erfindungsgemäße Polyurethane PU, die zu einer Erhöhung der dynamischen Viskosität von Wasser enthaltenden Zubereitungen führen. Sie können als eine alternative Lösung der sich stellenden Aufgabe - Modifizierung der rheologischen Eigenschaften von Wasser enthaltenden Zubereitungen - zu Verdickungsmitteln aus dem Stand der Technik gesehen werden.

Bevorzugt sind Polyurethane PU, deren 10-gewichtsprozentige wässrige Dispersionen eine dynamische Viskosität, gemessen wie nachfolgend beschrieben bei einer Schergeschwindigkeit von 100 1/s, von mindestens 100 mPa*s, besonders bevorzugt von mindestens 200 mPa*s und ganz besonders bevorzugt von mindestens 300 mPa*s aufweisen. Die wässrigen Dispersionen der erfindungsgemäßen Polyurethane PU können dabei entweder newtonsches oder aber strukturviskoses Verhalten zeigen. Strukturviskose Dispersionen, welche die erfindungsgemäßen Polyurethane PU enthalten, weisen bevorzugt dynamische Viskositäten von mindestens 1000 mPa*s, besonders bevorzugt sogar von mindestens 3000 mPa*s auf (10 Gew.-%ige wässrige Dispersionen, gemessen wie nachfolgend beschrieben bei einer Schergeschwindigkeit von 100 1/s).

Dem Fachmann ist bekannt, dass in Wasser enthaltenden Zubereitungen viele Verdickungsmittel ihre Wirkung einbüssen, d.h. die Viskosität der Zubereitung sinkt, sobald die Zubereitungen ebenfalls Salz und / oder Tensid enthalten. Dahingegen führen die erfindungsgemäßen Polyurethane PU in einer bevorzugten Ausführungsform zu einer Stabilisierung der Viskosität von Wasser enthaltenden Zubereitungen auch bei Zugabe von Salz und /oder Tensid. Besonders bevorzugt sind erfindungsgemäße Polyurethane PU, die bei einer Salzkonzentration von größer oder gleich 0,5 Gew.-% nach Zugabe zu einer Stabilisierung der dynamischen Viskosität, gemessen wie nachfolgend beschrieben, von Wasser enthaltenden Zubereitungen führen. Besonders bevorzugt sind solche Polyurethane, die zu einer Stabilisierung der dynamischen Viskosität bei Zugabe von größer gleich 0,5 Gew.-% Salz und Zugabe von größer gleich 1 Gew.-% Tensid führen, wobei die Reihenfolge der Zugaben gewünschtenfalls unerheblich ist.

In einer weiteren Ausführungsform wird die Viskosität von Wasser enthaltenden Zubereitungen, die mindestens ein Salz enthalten, durch das Vorhandensein der erfindungsgemäßen Polyurethane PU in der Zubereitung im Vergleich zu Zubereitungen, die nur Salz oder nur erfindungsgemäße Polyurethane PU enthalten, gesteigert. Dabei ist es die Reihenfolge unwesentlich, in der erfindungsgemäße Polyurethane PU und Salz zugegeben werden. Besonders bevorzugt sind erfindungsgemäße Polyurethane PU, die zu einer Erhöhung der dynamischen Viskosität, gemessen wie nachfolgend beschrieben, von Wasser enthaltenden Zubereitungen führen, wenn mindestens ein Salz oder mindestens ein Tensid oder Mischungen derselben in den Zubereitungen enthalten sind. Insbesondere bevorzugt sind erfindungsgemäße Polyurethane PU, die bei einer Salzkonzentration von größer oder gleich 0,5 Gew.-% zu einer Erhöhung der dynamischen Viskosität, gemessen wie nachfolgend beschrieben, von Wasser enthaltenden Zubereitungen führen. Besonders bevorzugt sind solche Polyurethane, die zu einer Steigerung der dynamischen Viskosität im Vergleich zu Zubereitungen, die weniger als 0,5 Gew.-%, bevorzugt 0,1 Gew.-%, Salz, oder weniger als 1 Gew.-%, bevorzugt 0,5 Gew.-% Tensid enthalten, führen.

Ganz besonders bevorzugt sind erfindungsgemäße Polyurethane PU, die bei einer Salzkonzentration von größer oder gleich 0,05 Gew.-% zu einer Erhöhung der dynamischen Viskosität, gemessen wie nachfolgend beschrieben, von Wasser enthaltenden Zubereitungen führen. Besonders bevorzugt sind solche Polyurethane, die zu einer Steigerung der dynamischen Viskosität im Vergleich zu Zubereitungen, die weniger als 0,05 Gew.-%, bevorzugt kleiner gleich 0,01 Gew.-%, Salz, oder weniger als 0,5 Gew.-%, bevorzugt kleiner gleich 0,1 Gew.-% Tensid enthalten, führen.

Ein weiterer Vorteil der erfindungsgemäßen Polyurethane ist die Mizellenbildung in Wasser. Die kritische Stoffkonzentration zur Mizellenbildung, auch kritische Mizellenkonzentration (im Englischen "Critical Micelle Concentration" CMC) genannt, gibt die Konzentration eines Stoffes, meist eines Stoffes der hydrophobe und hydrophile Abschnitte innehat, an, bei der spontan Mizellen gebildet werden. Die CMC der erfindungsgemäßen Polyurethane in Wasser, ermittelt wie nachfolgend beschrieben, ist bevorzugt kleiner gleich 1 g/L, besonders bevorzugt kleiner gleich 0,5 g/L, insbesondere bevorzugt kleiner gleich 0,25 g/L und ganz besonders bevorzugt kleiner gleich 0,1 g/L.

Ein weiterer Vorteil der erfindungsgemäßen Polyurethane, der erfindungsgemäßen Verfahren zu Ihrer Herstellung und der erfindungsgemäßen Zubereitungen ist die bevorzugte Verwendung von Zink- und/oder Titanhaltigen Katalysatoren bei der Herstellung der Polyurethane PU. Gerade im Bereich von kosmetischen Zubereitungen sind die aus dem Stand der Technik bekannten Verfahren mit Zinn nicht länger erwünscht, da auch in den Produkten und daraus resultierenden Zubereitungen Zinn enthalten sein kann. Zinkhaltige Zusatzstoffe von kosmetischen Zubereitungen sind akzeptiert, wobei Zink ggfs. durch seine antibakteriellen und entzündungshemmenden Eigenschaften zusätzliche Vorteile einbringen kann.

Die erfindungsgemäßen Polyurethane PU können aufgrund ihrer Toleranz gegenüber hohen Salz- und gleichzeitig hohen Tensidgehalten auch bei extremen pH-Werten vorteilhaft auch in Home-Care-Zubereitungen wie beispielsweise Flüssigreinigungsmitteln als Verdicker verwendet werden.

Insbesondere sind die erfindungsgemäßen Polyurethane PU auch als Rheologiemodizierungsmittel für wasserstoffperoxidhaltige Zubereitungen hervorragend geeignet.

Die Erfindung wird anhand der folgenden nicht einschränkenden Beispiele näher erläutert.

Beispiele

Soweit nichts anderes angegeben ist, beziehen sich alle Prozentangaben auf Gewichtsprozent.
Bestimmung der dynamischen Viskosität
Die dynamischen Viskositäten der erfindungsgemäßen Polyurethane PU in wässriger Dispersion wurden in Form einer 10 gewichtsprozentigen Dispersion bei 23 °C gemessen. In den unten aufgeführten Beispielen wurde dazu stets die dynamische Viskosität bei Schergeschwindigkeiten von 100 1/s und 350 1/s bestimmt. Diese beiden Werte erlauben eine Aussage zu treffen, ob die erfindungsgemäßen Polyurethane PU in wässriger Dispersion strukturviskoses oder newtonsches Verdickerverhalten zeigen. Zur Bestimmung der dynamischen Viskosität nach DIN53019 wurde verwendet:

- Verwendetes Gerät: Rotationsviskosimeter Physica Rheolab MCI Portable von der Firma Anton Paar;
- Zylinder Messsystem, Z4 DIN Zylinder (Durchmesser 14 mm)
- Verwendetes Gerät: Rotationsviskosimeter Physica Rheolab MCI Portable von der Firma Anton Paar;
- Zylinder Messsystem, Z4 DIN Zylinder (Durchmesser 14 mm)

Synthesebeispiel 1: Herstellung von Polyurethanen PU.1

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 140 ppm betrug.
Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern. Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einem Isocyanat-Gehalt von 0,27 Gew.-% reagieren gelassen. Dann wurde ein Gemisch aus 1,42 kg eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO10 von der Firma BASF SE), und 1,64 kg eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.
Das entstandene Produkt PU.1 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.1 typischerweise 1 zu 12,4 bzw. 1 zu 13,6. Das letztere Verhältnis ergibt sich für Abschnitte S, die aus 10 Ethylenoxidresten bestehen, ersteres für solche, die aus 11 Ethylenoxidresten aufgebaut sind.
Das molare Verhältnis von Abschnitten P zu D beträgt 1:1,75.
Das Produkt PU.1 wurde in 86,73 kg Wasser dispergiert und auf Raumtemperatur (25 °C) abgekühlt Die Mischung aus Polymeren PU.1 (Mn = 17600 g/mol; Mw = 30500 g/mol) lag in Form einer wässrigen Dispersion vor, welche einen Feststoffgehalt von 20,5 Gew.-% aufwies. Die Viskosität einer 10 gewichtsprozentigen wässrigen Dispersion der Polyetherpolyurethane PU.1 bei 23 °C betrug 7700 mPa*s (Scherrate 100 1/s) bzw. 5900 mPa*s (Scherrate 350 1/s) und zeigte schwach strukturviskoses Verhalten.

Synthesebeispiel 2: Herstellung von Polyurethanen PU.2

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 250 ppm betrug.
Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern.
Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einen Isocyanat-Gehalt von 0,29 Gew.-% reagieren gelassen.
Dann wurde ein Gemisch aus 0,95 kg eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO1 0 von der Firma BASF SE), und 2,19 kg eines nicht-ionischen ethoxylierten Fettalkohols, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt und die Reaktionsmischung solange weiter auf 50 °C erhitzt, bis der Isocyanat-Gehalt 0 Gew.-% betrug.

Das Lösungsmittel Xylol wurde nachfolgend durch Vakuumdestillation bei erhöhter Temperatur bis zu einem Restgehalt von unter 500 ppm entfernt.

Das entstandene Produkt PU.2 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.2 typischerweise 1 zu 12,4 bzw. 1 zu 13,6. Das letztere Verhältnis ergibt sich für Abschnitte S, die aus 10 Ethylenoxidresten bestehen, ersteres für solche, die aus 11 Ethylenoxidresten aufgebaut sind.

Das molare Verhältnis von Abschnitten P zu D beträgt 1:1,75.

Das Produkt PU.2 wurde in 87,02 kg Wasser dispergiert und auf Raumtemperatur (25 °C) abgekühlt. Die Polymermischung PU.2 ($M_n$ = 16700 g/mol; $M_w$ = 29500 g/mol) lag in Form einer wässrigen Dispersion vor, welche einen Feststoffgehalt von 20,0 Gew.-% aufwies. Die Viskosität einer 10 gewichtsprozentigen wässrigen Dispersion der Polyetherpolyurethane PU.2 bei 23 °C betrug 26200 mPa*s (Scherrate 100 1/s) bzw. 12800 mPa*s (Scherrate 350 1/s) und zeigte ausgeprägt strukturviskoses Verhalten.

Synthesebeispiel 3: Herstellung von Polyurethanen PU.3

120,00 g eines linearen Polyethylenglykols mit einem zahlenmittleren Molekulargewicht von 6000 g/mol (z.B. Pluriol® E6000 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend weniger als 300 ppm betrug.

Nun wurde die Polymerlösung auf 50 °C abgekühlt. Durch Zugabe von 42 mg Zink-neodecanoat, gelöst in einem Gemisch aus aliphatischen Kohlenwasserstoffen, und 5,88 g Hexamethylendiisocyanat, gelöst in Xylol, wurde die Polymerisation gestartet und der Ansatz bei 50 °C bis zu einen Isocyanat-Gehalt von 0,25 Gew.-% reagieren gelassen.

Dann wurden 19,20 g eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO10 von der Firma BASF SE), gelöst in Xylol, hinzugefügt und die Reaktionsmischung solange weiter auf 50 °C erhitzt, bis der Isocyanat-Gehalt 0 Gew.-% betrug.

Das Lösungsmittel Xylol wurde nachfolgend durch Vakuumdestillation bei erhöhter Temperatur bis zu einem Restgehalt von unter 500 ppm entfernt.

Das entstandene Produkt PU.3 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.3 typischerweise 1 zu 13,6. Dieses Verhältnis ergibt sich für Abschnitte S, die aus 10 Ethylenoxidresten bestehen.

Das molare Verhältnis von Abschnitten P zu D beträgt 1:1,75.

Das Produkt PU.3 wurde in 580,3 g Wasser dispergiert und auf Raumtemperatur (25 °C) abgekühlt. Die Polymermischung PU.3 ($M_n$ = 27200 g/mol; $M_w$, = 51900 g/mol) lag in Form einer wässrigen Dispersion vor, welche einen Feststoffgehalt von 20,0 % aufwies. Die Viskosität einer 10 gewichtsprozentigen wässrigen Dispersion der Polyetherpolyurethane PU. 3 bei 23 °C betrug 680 mPa*s (Scherrate 100 1/s) bzw. 640 mPa*s (Scherrate 350 1/s) und zeigte newtonsches Verdickerverhalten.

Synthesebeispiel 4: Herstellung von Polyurethanen PU.4

17,75 kg eines linearen Polyethylenglykols mit einem zahlenmittleren Molekulargewicht von 6000 g/mol (z.B. Pluriol® E6000 von BASF SE) wurden in 23,50 kg Xylol unter Stickstoff gelöst. Nach dem Erhitzen der Lösung auf ca. 140 °C wurde Xylol abdestilliert, so dass der Wassergehalt des Reaktionsansatzes anschließend ca. 120 ppm betrug.

Nun wurde die Polymerlösung auf 50 °C abgekühlt und mit 13,1 g Essigsäure, gelöst in 500 ml Xylol, versetzt, um die zuvor quantitativ bestimmte Menge an Kaliumacetat im Polyethylenglykol abzupuffern.

Durch Zugabe von 37,28 g Zink-neodecanoat, gelöst in einem Gemisch aus aliphatischen Kohlenwasserstoffen und Xylol, und 870,0 g Hexamethylendiisocyanat, gelöst in Xylol, wurde die Polymerisation gestartet und der Ansatz bei 50 °C bis zu einen Isocyanat-Gehalt von 0,26 Gew.-% reagieren gelassen.

Dann wurden 2,84 kg eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO10 von der Firma BASF SE), gelöst in Xylol, hinzugefügt und die Reaktionsmischung solange weiter auf 50 °C erhitzt, bis der Isocyanat-Gehalt 0 Gew.-% betrug.

Das Lösungsmittel Xylol wurde nachfolgend durch Vakuumdestillation bei erhöhter Temperatur bis zu einem Restgehalt von unter 500 ppm entfernt.

Das entstandene Produkt PU.4 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.4 typischerweise 1 zu 13,6. Dieses Verhältnis ergibt sich für Abschnitte S, die aus 10 Ethylenoxidresten bestehen.

Das molare Verhältnis von Abschnitten P zu D beträgt 1:1,75.

Das Produkt PU.4 wurde in 85,84 kg Wasser dispergiert und auf Raumtemperatur (25 °C) abgekühlt. Die Polymermischung PU.4 (Mn = 19200 g/mol; Mw, = 30800 g/mol) lag in Form einer wässrigen Dispersion vor, welche einen Feststoffgehalt von 18,1 % aufwies. Die Viskosität einer 10 gewichtsprozentigen wässrigen Dispersion der Polyetherpolyurethane PU.4 bei 23 °C betrug 600 mPa*s (Scherrate 100 1/s) bzw. 570 mPa*s (Scherrate 350 1/s) und zeigte newtonsches Verdickerverhalten.

Synthesebeispiel 5: Herstellung von Polyurethanen PU.5

240,00 g eines linearen Polyethylenglykols mit einem Molekulargewicht von 6000 g/mol (z.B. Pluriol® E6000 von BASF SE) wurden in 934,00 g Xylol unter Stickstoff gelöst. Nach dem Erhitzen der Lösung auf ca.140 °C wurde Xylol abdestilliert, so dass der Wassergehalt des Reaktionsansatzes anschließend weniger als 300 ppm betrug.

Nun wurde die Polymerlösung auf 50 °C abgekühlt. Durch Zugabe von 84 mg Zink-neodecanoat, gelöst in aliphatischen Kohlenwasserstoffen, und 11,76 g Hexamethylendiisocyanat, gelöst in Xylol, wurde die Polymerisation gestartet und der Ansatz bei 50 °C bis zu einem Isocyanat-Gehalt von 0,22 Gew.-% reagieren gelassen.

Dann wurden 20,70 g eines nicht-ionischen ethoxylierten Fettalkohols, hergestellt aus einem gesättigten C13 Alkohol und einem durchschnittlichen Ethoxylierungsgrad von 3 (z.B. Lutensol® AO3 von BASF SE), gelöst in Xylol, hinzugefügt und die Reaktionsmischung solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt und der Rückstand anschließend in 1089,8 g Wasser dispergiert.

Das Verhältnis der Molekulargewichte eines hydrophilen Abschnittes S zu dem Molekulargewicht eines hydrophilen Abschnittes P beträgt in den Polyurethanen PU.5 typischerweise 1 zu 45,5. Dieses Verhältnis ergibt sich für die Abschnitte S, die aus 3 Ethylenoxidresten bestehen.

Das molare Verhältnis von Abschnitten P zu D beträgt 1:1,75.

Nach dem Abkühlen auf Raumtemperatur (25 °C) lagen die Polymere PU.5 (Mn = 21300 g/mol; Mw = 36300 g/mol) in Form einer wässrigen Dispersion vor, welche einen Feststoffgehalt von 20,1 Gew.-% aufwies. Die Viskosität einer 10 gewichtsprozentigen wässrigen Dispersion der Polyetherpolyurethane PU.5 bei 23 °C betrug 10900 mPa*s (Scherrate 100 1/s) bzw. 9200 mPa*s (Scherrate 350 1/s) und zeigte schwach strukturviskoses Verhalten.

Synthesebeispiel 6: Herstellung von Polyurethanen PU.6

180,00 g eines linearen Polyethylenglykols mit einem Molekulargewicht von 6000 g/mol (z.B. Pluriol® E6000 von BASF SE) wurden in 180,00 g Aceton unter Stickstoff gelöst. Nach dem Erhitzen der Lösung auf Rückfluss (ca. 56 °C Innentemperatur) wurden kontinuierlich weitere 1362,4 g Aceton zugegeben und gleichzeitig insgesamt 1362,4 g Aceton abdestilliert. Der Wassergehalt des Reaktionsansatzes betrug anschließend nur noch ca. 240 ppm.

Nun wurde die Polymerlösung auf 50 °C abgekühlt. Durch Zugabe von 189 mg Zink-neodecanoat, gelöst in aliphatischen Kohlenwasserstoffen, und 8,82 g Hexamethylendiisocyanat, gelöst in Aceton, wurde die Polymerisation gestartet und der Ansatz bei 50 °C bis zu einem Isocyanat-Gehalt von 0,33 Gew.-% reagieren gelassen.

Dann wurden 15,53 g eines nicht-ionischen ethoxylierten Fettalkohols, hergestellt aus einem gesättigten C13 Alkohol und einem durchschnittlichen Ethoxylierungsgrad von 3 (z.B. Lutensol® AO3 von BASFSE), gelöst in Aceton, hinzugefügt und die Reaktionsmischung solange weiter auf 50 °C erhitzt, bis der Isocyanat-Gehalt 0 Gew.-% betrug. Das Lösungsmittel Aceton wurde nachfolgend durch Vakuumdestillation bis zu einem Restgehalt von unter 500 ppm entfernt und der Rückstand in 817,4 g Wasser dispergiert.

Das Verhältnis der Molekulargewichte eines hydrophilen Abschnittes S zu dem Molekulargewicht eines hydrophilen Abschnittes P beträgt in den Polyurethanen PU.6 typischerweise 1 zu 45,5. Dieses Verhältnis ergibt sich für die Abschnitte S, die aus 3 Ethylenoxidresten bestehen.

Das molare Verhältnis von Abschnitten P zu D beträgt 1:1,75.

Nach dem Abkühlen auf Raumtemperatur (25 °C) lagen die Polymere PU.6 (Mn = 24900 g/mol; Mw = 40000 g/mol) in Form einer wässrigen Dispersion vor, welche einen Feststoffgehalt von 19,6 Gew.-% aufwies. Die Viskosität einer 10 gewichtsprozentigen wässrigen Dispersion der Polyetherpolyurethane PU.6 bei 23 °C betrug 8800 mPa*s (Scherrate 100 1/s) bzw. 7800 mPa*s (Scherrate 350 1/s) und zeigte schwach strukturviskoses Verhalten.

Synthesebeispiel 7: Herstellung von Polyurethanen PU.7

120,00 g eines linearen Polyethylenglykols mit einem zahlenmittleren Molekulargewicht von 6000 g/mol (z.B.

Pluriol® E6000 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 120 ppm betrug.

Nun wurde die Polymerlösung auf 50 °C abgekühlt und mit 107 mg Essigsäure, gelöst in 5 ml Xylol, versetzt, um die zuvor quantitativ bestimmte Menge an Kaliumacetat im Polyethylenglykol abzupuffern. Durch Zugabe von 252 mg Zinkneodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,25 Gew.-% reagieren gelassen. Dann wurden 22,20 g eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.7 ist eine Mischung, welche lineare Polyurethane mit randständigen, unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.7 typischerweise 1 zu 12,4. Dieses Verhältnis ergibt sich für Abschnitte S, die aus 11 Ethylenoxidresten bestehen.

Das molare Verhältnis von Abschnitten P zu D beträgt 1:1,75.

Das Produkt PU.7 wurde in 592,3 g Wasser dispergiert und auf Raumtemperatur (25 °C) abgekühlt. Die Mischung aus Polymeren PU.7 (Mn = 18700 g/mol; Mw = 30900 g/mol) lag in Form einer wässrigen Dispersion vor, welche einen Feststoffgehalt von 20,4 Gew.-% aufwies. Die Viskosität einer 10 gewichtsprozentigen wässrigen Dispersion der Polyetherpolyurethane PU.7 bei 23 °C betrug 35500 mPa*s (Scherrate 100 1/s) bzw. 14500 mPa*s (Scherrate 350 1/s) und zeigte stark strukturviskoses Verhalten.

Synthesebeispiel 8: Herstellung von Polyurethanen PU.8

180,00 g eines linearen Polyethylenglykols mit einem zahlenmittleren Molekulargewicht von 9000 g/mol (z.B. Pluriol® E9000 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 70 ppm betrug.

Nun wurde die Polymerlösung auf 50 °C abgekühlt und mit 208 mg Essigsäure, gelöst in 5 ml Xylol, versetzt, um die zuvor quantitativ bestimmte Menge an Kaliumacetat im Polyethylenglykol abzupuffern. Durch Zugabe von 378 mg Zinkneodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,27 Gew.-% reagieren gelassen. Dann wurden 10,20 g eines nicht-ionischen ethoxylierten Fettalkohols, hergestellt aus einem gesättigten iso- C13 Alkohol und einem durchschnittlichen Ethoxylierungsgrad von 3 (z.B. Lutensol® TO3 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.8 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.8 typischerweise 1 zu 68,2. Dieses Verhältnis ergibt sich für Abschnitte S, die aus 3 Ethylenoxidresten bestehen.

Das molare Verhältnis von Abschnitten P zu D beträgt 1:1,75.

Das Produkt PU.8 wurde in 784,3 g Wasser dispergiert und auf Raumtemperatur (25 °C) abgekühlt. Die Mischung aus Polymeren PU.8 (Mn = 27300 g/mol; Mw = 46500 g/mol) lag in Form einer wässrigen Dispersion vor, welche einen Feststoffgehalt von 20,2 Gew.-% aufwies. Die Viskosität einer 10 gewichtsprozentigen wässrigen Dispersion der Polyetherpolyurethane PU.8 bei 23 °C betrug 1060 mPa*s (Scherrate 100 1/s & Scherrate 350 1/s) und zeigte ausgeprägtes, newtonsches Verhalten.

Synthesebeispiel 9: Herstellung von Polyurethanen PU.9

180,00 g eines linearen Polyethylenglykols mit einem zahlenmittleren Molekulargewicht von 9000 g/mol (z.B. Pluriol® E9000 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 70 ppm betrug.

Nun wurde die Polymerlösung auf 50 °C abgekühlt und mit 208 mg Essigsäure, gelöst in 5 ml Xylol, versetzt, um die zuvor quantitativ bestimmte Menge an Kaliumacetat im Polyethylenglykol abzupuffern. Durch Zugabe von 378 mg Zinkneodecanoat, gelöst in einem Gemisch aus aliphatischen Kohlenwasserstoffen und Xylol, und 5,88 g Hexamethylendiisocyanat, gelöst in Xylol, wurde die Polymerisation gestartet und der Ansatz bei 50 °C bis zu einem Isocyanat-Gehalt von 0,28 Gew.-% reagieren gelassen. Dann wurde ein Gemisch aus 5,10 g eines nicht-ionischen ethoxylierten Fettalkohols, hergestellt aus einem gesättigten iso- C13 Alkohol und einem durchschnittlichen Ethoxylierungsgrad von 3 (z.B. Lutensol® TO3 von der Firma BASF SE), und 11,10 g eines nicht-ionischen ethoxylierten Fettalkoholgemischs, hergestellt aus einem gesättigten C16/C18 Alkoholgemisch und einem durchschnittlichen Ethoxylierungsgrad von 11 (z.B. Lutensol® AT11 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.9 ist eine Mischung, welche lineare Polyurethane mit randständigen verzweigten und/oder unverzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.9 typischerweise 1 zu 12,4 oder 1 zu 68,2. Das letztgenannte Verhältnis ergibt sich für Abschnitte S, die aus 3 Ethylenoxidresten bestehen, ersteres für solche, die aus 11 Ethylenoxidresten aufgebaut sind.
Das molare Verhältnis von Abschnitten P zu D beträgt 1:1,75.
Das Produkt PU.9 wurde in 764,0 g Wasser dispergiert und auf Raumtemperatur (25 °C) abgekühlt Die Mischung aus Polymeren PU.9 ($M_n$ = 25000 g/mol; $M_w$ = 45500 g/mol) lag in Form einer wässrigen Dispersion vor, welche einen Feststoffgehalt von 20,8 Gew.-% aufwies. Die Viskosität einer 10 gewichtsprozentigen wässrigen Dispersion der Polyetherpolyurethane PU.9 bei 23 °C betrug 7500 mPa*s (Scherrate 100 1/s) bzw. 4500 mPa*s (Scherrate 350 1/s) und zeigte stark strukturviskoses Verhalten.

Synthesebeispiel 10: Herstellung von Polyurethanen PU.10

120,00 g eines linearen Polyethylenglykols mit einem zahlenmittleren Molekulargewicht von 1500 g/mol (z.B. Pluriol® E1500 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 110 ppm betrug.
Nun wurde die Polymerlösung auf 50 °C abgekühlt und mit 90 mg Essigsäure, gelöst in 5 ml Xylol, versetzt, um die zuvor quantitativ bestimmte Menge an Kaliumacetat im Polyethylenglykol abzupuffern. Durch Zugabe von 252 mg Zinkneodecanoat, gelöst in einem Gemisch aus aliphatischen Kohlenwasserstoffen und Xylol, und 15,72 g Hexamethylendiisocyanat, gelöst in Xylol, wurde die Polymerisation gestartet und der Ansatz bei 50 °C bis zu einem Isocyanat-Gehalt von 0,29 Gew.-% reagieren gelassen. Dann wurden 17,41 g eines nicht-ionischen ethoxylierten Fettalkohols, hergestellt aus einem gesättigten iso- C13 Alkohol und einem durchschnittlichen Ethoxylierungsgrad von 10 (z.B. Lutensol® TO1 0 von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.
Das entstandene Produkt PU.10 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.10 typischerweise 1 zu 13,6. Dieses Verhältnis ergibt sich für Abschnitte S, die aus 10 Ethylenoxidresten bestehen.
Das molare Verhältnis von Abschnitten P zu D beträgt 1:1,17.
Das Produkt PU.10 wurde in 612,5 g Wasser dispergiert und auf Raumtemperatur (25 °C) abgekühlt Die Mischung aus Polymeren PU.10 ($M_n$ = 18600 g/mol; $M_w$ = 34900 g/mol) lag in Form einer wässrigen Dispersion vor, welche einen Feststoffgehalt von 20,1 Gew.-% aufwies. Die Viskosität einer 10 gewichtsprozentigen wässrigen Dispersion der Polyetherpolyurethane PU.10 bei 23 °C betrug 165 mPa*s (Scherrate 100 1/s & Scherrate 350 1/s) und zeigte ausgeprägtes, newtonsches Verhalten.

Synthesebeispiel 11: Herstellung von Polyurethanen PU.11

90,00 g eines linearen Polyethylenglykols mit einem zahlenmittleren Molekulargewicht von 1500 g/mol (z.B. Pluriol® E1500 von BASF SE) wurden in 467,00 g Xylol unter Stickstoff gelöst. Nach dem Erhitzen der Lösung auf ca. 140 °C wurde Xylol abdestilliert, so dass der Wassergehalt des Reaktionsansatzes anschließend nur noch ca. 80 ppm betrug.
Nun wurde die Polymerlösung auf 50 °C abgekühlt und mit 68 mg Essigsäure, gelöst in 5 ml Xylol, versetzt, um die zuvor quantitativ bestimmte Menge an Kaliumacetat im Polyethylenglykol abzupuffern. Durch Zugabe von 189 mg Zinkneodecanoat, gelöst in einem Gemisch aus aliphatischen Kohlenwasserstoffen und Xylol, und 17,64 g Hexamethylendiisocyanat, gelöst in Xylol, wurde die Polymerisation gestartet und der Ansatz bei 50 °C bis zu einem Isocyanat-Gehalt von 0,97 Gew.-% reagieren gelassen. Dann wurden 99,00 g eines nicht-ionischen ethoxylierten Fettalkohols, hergestellt aus einem gesättigten iso- C13 Alkohol und einem durchschnittlichen Ethoxylierungsgrad von 20 (z.B. Lutensol® TO20

16

von der Firma BASF SE), gelöst in Xylol, hinzugefügt. Die Reaktionsmischung wurde solange weiter auf 50 °C erhitzt, bis der Isocyanat-Gehalt 0 Gew.-% betrug. Das Lösungsmittel Xylol wurde nachfolgend durch Vakuumdestillation bei erhöhter Temperatur bis zu einem Restgehalt von unter 500 ppm entfernt.

Das entstandene Produkt PU.11 ist eine Mischung, welche lineare Polyurethane mit randständigen verzweigten Abschnitten T enthält. Das Verhältnis der Molekulargewichte eines hydrophilen Abschnittes S zu dem Molekulargewicht eines hydrophilen Abschnittes P beträgt in den Polyurethanen PU.11 typischerweise 1 zu 1,7. Dieses Verhältnis ergibt sich für Abschnitte S, die aus 20 Ethylenoxidresten bestehen.

Das molare Verhältnis von Abschnitten P zu D beträgt 1:1,75.

Das Produkt PU.11 wurde in 826,6 g Wasser dispergiert und auf Raumtemperatur (25 °C) abgekühlt Die Mischung aus Polymeren PU.11 (Mn = 4000 g/mol; Mw = 9000 g/mol) lag in Form einer wässrigen Dispersion vor, welche einen Feststoffgehalt von 20,0 Gew.-% aufwies. Die Viskosität einer 10 gewichtsprozentigen wässrigen Dispersion der Polyetherpolyurethane PU.11 bei 23 °C betrug 150 mPa*s (Scherrate 100 1/s & Scherrate 350 1/s) und zeigte ausgeprägtes, strukturviskoses Verhalten.

Bestimmung der kritischen Mizellenkonzentration

Die CMC der erfindungsgemäßen Polyurethane in Wasser wurde mit der Methode der dynamischen Lichtstreuung ermittelt.

Hierzu wurde ein Goniometer SP - 86 (ALV-Laser Vertriebsgesellschaft mbH, Langen, Germany) als kombinierte DLS/SLS Anlage verwendet. Die Anlage umfasste auch ein ALV 5000 Korrelator und einen He-Ne Laser der Wellenlänge 633 nm (beide ebenfalls ALV, Langen). Die verwendeten Bedingungen für die Messreihen umfassend Konzentrationen von 0,0001 g/L bis 10 g/L waren ein Messwinkel von 90° bei einer Temperatur von 23°C. Die Auswertung erfolgte mithilfe des im Stand der Technik bekannten Programms CONTIN (Constrained Inversion) mit Intensitätsverteilung (CONTIN ebenfalls von ALV, Langen).

Vergleichsbeispiel:

Ein nicht-ionisches, hydrophob modifiziertes, ethoxyliertes Urethan des Standes der Technik, das aus Stearylalkohol, einem Diisocyanat und einem Polyethylenglykol hergestellt wird (vertrieben von der Firma Rohm & Haas als Aculyn® 46) wurde zur Bestimmung der CMC im Vergleich eingesetzt. Aculyn® 46 wies keine messbare CMC auf. Bei Konzentrationen von 0,001 bis 10 g/L lagen als Hauptkomponente stets größere undefinierte Aggregate im Bereich 100 bis 500 nm vor.

CMC der Polyurethane der vorliegenden Erfindung:

Für die in Synthesebeispiel 1 und 2 hergestellten Mischungen von Polyurethanen PU.1 sowie PU.2 wurde gefunden, dass bei 0,1 g/L definierte Mizellen mit mittleren Teilchendurchmessern von 30 nm vorlagen. Die CMC betrug daher für beide kleiner 0,1 g/L. Für die in Synthesebeispiel 4 hergestellten erfindungsgemäßen Polyurethane PU.4 wurde gefunden, dass bei einer Konzentration von PU.4 von 1 g/L Mizellen mit Durchmesser 17 nm vorlagen und bei einer Konzentration von 0,1 g/L sowohl Mizellen einer mittleren Größe von 15 nm als auch ein geringer Anteil undefinierte Aggregate einer Größe von ungefähr. 200 nm nebeneinander existierten. Daher lag auch in diesem Fall eine CMC von <0,1 g/L vor.

Zubereitungsbeispiel 1: Herstellung kosmetischer Zubereitungen unter Verwendung der Polyurethane PU.1. bis PU.5 mit einer nicht-ionischen Grundlage (Z.1.1 bis Z.1.5)

Die kosmetischen Zubereitungen wurden durch Zugabe der Wasserphase B zur der Ölphase A und anschließendem Versetzen der erhaltenen O/W-Emulsion mit dem Konservierungsmittel (Phase C) hergestellt. Man erhielt die auf einer nicht-ionischen Grundlage basierenden Zubereitungen Z.1.1 bis Z.1.5. (Tab. 1).

Tabelle 1. Zusammensetzung der auf einer nicht-ionischen Grundlage basierenden kosmetischen Zubereitungen Z.1.1 bis Z.1.5.

| Phase | Inhaltsstoffe | Z.1.1 | Z.1.2 | Z.1.3 | Z.1.4 | Z.1.5 |
|---|---|---|---|---|---|---|
| Phase A | Ceteareth-6, Stearyl Alkohol | 2,0 g | 2,0 g | 2,0 g | 2,0 g | 2,0 g |
| | Ceteareth-25 | 2,0 g | 2,0 g | 2,0 g | 2,0 g | 2,0 g |
| | Cetearyl Alkohol | 2,5 g | 2,5 g | 2,5 g | 2,5 g | 2,5 g |
| | Paraffinöl | 5,0 g | 5,0 g | 5,0 g | 5,0 g | 5,0 g |
| | Cetearyl Ethylhexanoat | 5,0 g | 5,0 g | 5,0 g | 5,0 g | 5,0 g |

(fortgesetzt)

| Phase | Inhaltsstoffe | Z.1.1 | Z.1.2 | Z.1.3 | Z.1.4 | Z.1.5 |
|---|---|---|---|---|---|---|
| Phase B | PU | PU.1 0,5 g | PU.2 0,5 g | PU.3 2,0 g | PU.4 2,0 g | PU.5 0,5 g |
| | 1,2-Propylenglykol | 5,0 g | 5,0 g | 5,0 g | 5,0 g | 5,0 g |
| | Wasser | 77,5 g | 77,5 g | 76,0 g | 76,0 g | 77,5 g |
| Phase C | Konservierungsmittel Euxyl® K300 (Phenoxyethanol, Methylparaben, Ethylparaben, Butylparaben, Propylparaben, Isobutylparaben), kommerziell erhältlich von Fischer-Chemie, Wiesbaden | 0,5 g | 0,5 g | 0,5 g | 0,5 g | 0,5 g |

Zubereitungsbeispiel 2: Herstellung kosmetischer Zubereitungen unter Verwendung der Polyurethane PU.1. bis PU.5; nicht-ionische Grundlage (Z.2.1 bis Z.2.5)

Die kosmetischen Zubereitungen wurden durch Zugabe der Wasserphase B zur Ölphase A und anschließendem Versetzen der erhaltenen O/W-Emulsion mit dem Konservierungsmittel (Phase C) hergestellt. Man erhielt die auf einer nicht-ionischen Grundlage basierenden Zubereitungen Z.2.1 - Z.2.5. (Tab. 2).

Tabelle 2. Zusammensetzung der auf einer nicht-ionischen Grundlage basierenden kosmetischen Zubereitungen Z. 2.1 - Z.2.5.

| Phase | Inhaltsstoffe | Z.2.1 | Z.2.2 | Z.2.3 | Z.2.4 | Z.2.5 |
|---|---|---|---|---|---|---|
| Phase A | Glyceryl Stearat | 2,0 g | 2,0 g | 2,0 g | 2,0 g | 2,0 g |
| | Stearyl Alkohol | 2,0 g | 2,0 g | 2,0 g | 2,0 g | 2,0 g |
| | Cyclopentasiloxan, Cyclohexasiloxan | 3,0 g | 3,0 g | 3,0 g | 3,0 g | 3,0 g |
| | Dicaprylyl Ether | 3,0 g | 3,0 g | 3,0 g | 3,0 g | 3,0 g |
| | Dimethicon | 2,0 g | 2,0 g | 2,0 g | 2,0 g | 2,0 g |
| | Aluminum Stärke Octenylsuccinat | 1,0 g | 1,0 g | 1,0 g | 1,0 g | 1,0 g |
| | PEG-40 Stearat | 2,0 g | 2,0 g | 2,0 g | 2,0 g | 2,0 g |
| Phase B | PU | PU.1 0,5 g | PU.2 0,5 g | PU.3 2,0 g | PU.4 2,0 g | PU.5 0,5 g |
| | Glycerin | 5,0 g | 5,0 g | 5,0 g | 5,0 g | 5,0 g |
| | Wasser | 79,0 g | 79,0 g | 77,5 g | 77,5 g | 79,0 g |

(fortgesetzt)

| Phase | Inhaltsstoffe | Z.2.1 | Z.2.2 | Z.2.3 | Z.2.4 | Z.2.5 |
|---|---|---|---|---|---|---|
| Phase C | Konservierungsmittel Euxyl® K300 (Phenoxyethanol, Methylparaben, Ethylparaben, Butylparaben, Propylparaben, Isobutylparaben), kommerziell erhältlich von Fischer-Chemie, Wiesbaden | 0,5 g | 0,5 g | 0,5 g | 0,5 g | 0,5 g |

Bestimmung der dynamischen Viskosität von Zubereitungen mit Hilfsstoffen Die dynamische Viskosität von Wasser enthaltenden Zubereitungen, die weitere Hilfsstoffe enthalten, z.B. jener kosmetischer Zubereitungen, die nicht einschränkend in den Zubereitungssbeispielen offenbart sind, wurde mit Hilfe eines Brookfield-Viskosimeters (Firma Brookfield), Typ DV-II+Pro Viskosimeter (Model: RVDVII+Pro) bestimmt. Als Messsystem wurde ein RV Spindelset bei einer Temperatur von 20 °C und 20 U/Min. Schergeschwindigkeit eingesetzt.

Viskositäten der kosmetischen Zubereitungen Z.1.1 bis Z.1.5 (basierend auf einer nicht-ionischen Grundlage) in Abhängigkeit von der Salzkonzentration

Tabelle 3. Viskositäten der kosmetischen Zubereitungen Z.1.1 bis Z.1.5 in Abhängigkeit von der Salzkonzentration.

| Zubereitung | Dynamische Viskosität [Pa*s] | | | | |
|---|---|---|---|---|---|
| | 0 Gew.-% NaCl | 0,5 Gew.-% NaCl | 2,0 Gew.-% NaCl | 5,0 Gew.-% NaCl | 10,0 Gew.-% NaCl |
| Z.1.1 | 33,2 | 24,0 | 13,2 | 7,9 | 7,0 |
| Z.1.2 | 39,5 | 29,8 | 14,8 | 11,0 | 11,3 |
| Z.1.3 | 4,1 | 6,1 | 6,3 | 7,7 | 8,6 |
| Z.1.4 | 3,0 | 4,3 | 3,9 | 4,3 | 2,4 |
| Z.1.5 | 11,3 | 9,7 | 6,9 | 5,1 | 3,8 |

Die Zubereitungen Z.1.3 und Z.1.4 zeigen bei Salzzugabe steigende bzw. weitgehend stabile Viskositäten. Z.1.1, Z.1.2 und Z.1.5 zeigen auch bei moderater Salzzugabe noch gute Verdickerleistung.

Viskositäten der kosmetischen Zubereitungen Z.2.1 bis Z.2.5 (basierend auf einer nicht-ionischen Grundlage) in Abhängigkeit von der Salzkonzentration

Tabelle 4. Viskositäten der kosmetischen Zubereitungen Z.2.1 bis Z.2.5 in Abhängigkeit von der Salzkonzentration.

| Zubereitung | Dynamische Viskosität [Pa*s] | | | | |
|---|---|---|---|---|---|
| | 0 Gew.-% NaCl | 0,5 Gew.-% NaCl | 2,0 Gew.-% NaCl | 5,0 Gew.-% NaCl | 10,0 Gew.-% NaCl |
| Z.2.1 | 23,3 | 18,0 | 15,0 | 10,6 | 5,3 |
| Z.2.2 | 16,4 | 11,2 | 9,5 | 7,6 | 4,6 |
| Z.2.3 | 13,1 | 14,4 | 15,6 | 18,0 | 20,3 |
| Z.2.4 | 5,4 | 13,0 | 13,3 | 15,2 | 13,7 |
| Z.2.5 | 27,0 | 30,6 | 23,5 | 23,8 | 16,1 |

Zubereitung Z.2.5 zeigt bei Salzzugabe stabile und teilweise sogar steigende Viskositäten. Dies ist für Z.2.3 und Z.2.4 noch ausgeprägter, diese zeigen eine starke Erhöhung der dynamischen Viskositäten bei Salzzugabe bis 10 Gew.-%.

Z.2.1 und Z.2.2 weisen auch bei moderater Salzzugabe noch gute Verdickerleistung auf.

**Patentansprüche**

1. In Wasser dispergierbares Polyurethan (PU) mit einem im Wesentlichen linearen Rückgrat aufgebaut aus alternierenden hydrophilen und hydrophoben Abschnitten, wobei

    a. die beiden endständigen Abschnitte (T) hydrophob sind,
    b. sich an jeden Abschnitt T je ein hydrophiler Abschnitt (S) direkt anschließt,
    c. sich an jeden Abschnitt S mindestens ein hydrophober Abschnitt (D) auf mindestens einer Seite direkt anschliesst, und
    d. wobei mindestens ein hydrophiler Abschnitt (P) enthalten ist, wobei mindestens ein hydrophober Abschnitt D zwei Abschnitte P trennt, falls mehr als ein Abschnitt P enthalten ist,

und das Polyurethan mindestens drei hydrophile Abschnitte umfasst, und das Verhältnis der Molekulargewichte eines jeden hydrophilen Abschnittes S zu dem Molekulargewicht eines jeden hydrophilen Abschnittes P von 1 zu 1,4 bis 1 zu 140 beträgt, die mindestens zwei hydrophoben Abschnitte D aliphatische Diisocyanatreste sind und der mindestens eine hydrophile Abschnitt P ein Polyetherrest mit einem zahlenmittleren Molekulargewicht von mindestens 1500 g/mol ist, oder eine Mischung unterschiedlicher Polyurethane PU.

2. Polyurethan nach Anspruch 1 worin mindestens einer der beiden hydrophoben endständigen Abschnitte T ein verzweigter Alkylrest ist.

3. Polyurethan nach Anspruch 1, das eine Mischung von Polyurethanen PU ist, deren endständige, hydrophobe Abschnitte T verzweigte oder unverzweigte Alkylreste sind.

4. Polyurethan nach mindestens einem der Ansprüche 1 bis 3 worin alle hydrophilen Abschnitte Polyetherreste sind.

5. Polyurethan nach mindestens einem der Ansprüche 1 bis 4 worin die mindestens zwei hydrophilen Abschnitte S Ethylenoxidreste sind.

6. Polyurethan nach mindestens einem der Ansprüche 1 bis 5 worin der mindestens eine hydrophile Abschnitt P ein zahlenmittleres Molekulargewicht von 1500 bis 10000 g/mol aufweist.

7. Verfahren zur Herstellung von Polyurethanen gemäß mindestens einem der Ansprüche 1 bis 6 **dadurch gekennzeichnet, dass** die Herstellung in Gegenwart von mindestens einem Zink-Carboxylat oder mindestens einem Titanalkoholat oder Mischungen derselben erfolgt.

8. Verfahren nach Anspruch 7 **dadurch gekennzeichnet, dass** die Herstellung in Gegenwart von mindestens einem Zink-Carboxylat erfolgt.

9. Verfahren nach Anspruch 7 oder 8 **dadurch gekennzeichnet, dass** man die Herstellung in zwei Stufen erfolgt.

10. Verfahren nach Anspruch 9 **dadurch gekennzeichnet, dass** man mindestens ein Polyetherdiol mit mindestens einem aliphatischen Diisocyanat und in Gegenwart mindestens eines Zink-Carboxylates oder mindestens eines Titanalkoholates oder Mischungen derselben umsetzt, in einem zweiten Schritt mit mindestens einem alkoxylierten Fettalkohol umsetzt, und danach aufarbeitet.

11. Verfahren nach mindestens einem der Ansprüche 7 bis 10 **dadurch gekennzeichnet, dass** das Verhältnis von eingesetzten Polyetherdiolen zu eingesetzten Diisocyanaten im Bereich von 1 zu 1,1 bis 1 zu 1,75 liegt.

12. Verwendung von Polyurethanen gemäß mindestens einem der Ansprüche 1 bis 6 oder hergestellt gemäß mindestens einem der Verfahren gemäß den Ansprüchen 7 bis 11 zur Herstellung von Zubereitungen, die Wasser enthalten.

13. Verwendung nach Anspruch 12 worin die Zubereitungen neben Wasser mindestens ein Salz oder mindestens ein Tensid oder Mischungen derselben enthalten.

14. Zubereitungen enthaltend mindestens ein Polyurethan gemäß mindestens einem der Ansprüche 1 bis 6 oder hergestellt gemäß mindestens einem der Verfahren gemäß den Ansprüchen 7 bis 11.

15. Zubereitungen nach Anspruch 14 **dadurch gekennzeichnet, dass** die Zubereitung eine wässrige Dispersion ist.

**Europäisches Patentamt**

**EUROPÄISCHER RECHERCHENBERICHT**

Nummer der Anmeldung

EP 08 15 5673

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | US 4 079 028 A (EMMONS WILLIAM D ET AL WILLIAM D EMMONS [US] ET AL) 14. März 1978 (1978-03-14) | 1-6, 12-15 | INV. C08G18/10 C08G18/28 C08G18/48 C09D7/00 |
| Y | * Spalte 4, Zeile 50 - Spalte 5, Zeile 10 * * Spalte 8, Absatz 48 - Spalte 8, Absatz 62 * * Beispiele 75-96 * * Ansprüche 1,6 * ----- | 7-11 | |
| X | EP 0 639 595 A (BAYER AG [DE]) 22. Februar 1995 (1995-02-22) | 1-6, 12-15 | |
| Y | * Seite 4, Zeile 47 - Seite 5, Zeile 5 * * Seite 5, Zeile 30 - Seite 5, Zeile 34 * * Beispiel 10 * * Ansprüche 1,3 * ----- | 7-11 | |
| X | EP 0 495 373 A (GOLDSCHMIDT AG TH [DE]) 22. Juli 1992 (1992-07-22) | 1-6, 12-15 | RECHERCHIERTE SACHGEBIETE (IPC) |
| Y | * Seite 5, Zeile 5 - Seite 6, Zeile 13 * * Seite 7, Zeile 1 - Seite 7, Zeile 53 * * Tabellen 1,2 * * Ansprüche 1,5,6,11 * ----- | 7-11 | C08G C09D |
| A | BARMAR M ET AL: "Investigating the effect of hydrophobic structural parameters on the thickening properties of HEUR associative copolymers" 1. März 2005 (2005-03-01), EUROPEAN POLYMER JOURNAL, PERGAMON PRESS LTD. OXFORD, GB, PAGE(S) 619 - 626 , XP004719840 ISSN: 0014-3057 * Zusammenfassung * ----- | 1-15 | |

Der vorliegende Recherchenbericht wurde für alle Patentansprüche erstellt

| Recherchenort | Abschlußdatum der Recherche | Prüfer |
|---|---|---|
| München | 25. September 2008 | Heidenhain, Frank |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 08 15 5673

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-09-2008

| Im Recherchenbericht angeführtes Patentdokument | Datum der Veröffentlichung | Mitglied(er) der Patentfamilie | Datum der Veröffentlichung |
|---|---|---|---|
| US 4079028 A | 14-03-1978 | KEINE | |
| EP 0639595 A | 22-02-1995 | AT 151789 T | 15-05-1997 |
| | | CA 2129932 A1 | 17-02-1995 |
| | | DE 4327481 A1 | 23-02-1995 |
| | | DK 639595 T3 | 06-10-1997 |
| | | ES 2102742 T3 | 01-08-1997 |
| | | JP 3364014 B2 | 08-01-2003 |
| | | JP 7076614 A | 20-03-1995 |
| | | US 5594087 A | 14-01-1997 |
| EP 0495373 A | 22-07-1992 | DE 4101239 C1 | 02-04-1992 |

EPO FORM P0461

Für nähere Einzelheiten zu diesem Anhang : siehe Amtsblatt des Europäischen Patentamts, Nr.12/82

23

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4079028 A
- US 4155892 A
- EP 1584331 A
- EP 1013264 A
- WO 20061002813 A
- EP 0725097 A

**In der Beschreibung aufgeführte Nicht-Patentliteratur**

- **von Blank et al.** *Progress in Organic Coatings,* 1999, vol. 35, 19 ff